# EUROPEAN PATENT APPLICATION

(11) **EP 4 837 924 A1**
(43) Date of publication of application: **11.11.2026**
(21) Application number: 24915243.0
(22) Date of filing: 31.12.2024
(51) Int. Cl.: C07K 16/46, C07K 16/28, C07K 16/32

(54) **MULTISPECIFIC MOLECULE TARGETING IL-11 AND TSLP**

(30) Priority: 02.01.2024 CN 202410009617
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: ZHAN, Yifan, Shanghai 201203 (CN); ZHU, Xiangyang, Shanghai 201203 (CN); YU, Haijia, Shanghai 201203 (CN); XU, Jingen, Shanghai 201203 (CN); ZHU, Xi, Shanghai 201203 (CN); CHEN, Shi, Shanghai 201203 (CN)
(74) Representative: Weickmann & Weickmann PartmbB
(86) International application number: PCT/CN2024/144114
(87) International publication number: WO 2025/146029

(57) **Abstract**

The present application relates to a multispecific molecule, comprising a first binding domain capable of targeting IL-11 and a second binding domain capable of targeting TSLP, the multispecific molecule being capable of treating a disease. The present application also provides the use of the multispecific molecule as a medicament.

## Description

### TECHNICAL FIELD

The present application relates to the field of biomedicine, and specifically to a multispecific molecule targeting IL-11 and TSLP, and uses thereof.

### BACKGROUND ART

Thymic stromal lymphopoietin (TSLP) is an interleukin-7 (IL-7)-like cytokine, a mature hTSLP is composed of 131 amino acid residues, and has a typical 4-stranded α-helix bundle structure. It is mainly produced in epithelial cells, smooth muscle cells, keratinocytes, stromal cells, fibroblasts, mast cells (MC), monocyte-macrophages, granulocytes, and dendritic cells (DC). As an important allergic factor secreted by epithelial cells, TSLP induces the onset of Th2 response in an organism by interacting with locally infiltrated DC cells and T cells. In addition, TSLP can also play an important coordinating and balancing role in the inflammatory response by its inhibitory effect on the Th1 pathway, causing the inflammatory response of the organism to shift from Th1 to Th2 direction.

TSLP receptor complex is a heterodimer composed of TSLP receptor (TSLPR) and IL-7 receptor α (IL-7Ra), both of which are highly expressed in myeloid dendritic cells. TSLP binds to TSLPR on the cell membrane, and then binds to IL-7Rα to form a stable TSLP-TSLPR-IL7Ra receptor complex, in which the intracellular segment of the TSLPR receptor recruits and activates JAK2, works together with JAK1 recruited by IL7Rα to activate downstream signaling molecule TSLP to bind to the surface receptor of myeloid dendritic cells, and then the dendritic cells secrete IL-8 and eotaxin-2 to recruit neutrophils and eosinophils, and secrete TARC and MDC to recruit Th2 cells. In addition, TSLP-activated DC cells induce differentiation of CD4+T cells to Th2 cells, which are capable of producing IL4, IL-5, IL-13 and TNF, and these cytokines promote the production of IgE, eosinophils and mucus to initiate allergic reactions, leading to diseases such as asthma, atopic dermatitis and the like.

Interleukin-11 (IL-11), discovered in 1990, is a member of the IL-6 family, comprises 178 amino acids, and has a molecular weight of about 19kDa. IL-11 can be expressed by many cell types, such as leukocytes, fibroblasts and epithelial cells, and its expression is regulated by a variety of cytokines, such as TGF-β, IL-1β, IL-22, IL-17F, IFN-γ, TNF-α, COX-2, etc. IL-11 activates the downstream signaling pathway by binding to IL-11 receptor α-subunit (IL-11Rα), and in turn forming a 2:2:2 (IL-11/IL-11Rα/GP130) complex with glycoprotein β receptor subunit (GP130).

The most well-studied physiological function of IL-11 is to promote the expansion of megakaryocytes, and in turn promote platelet production. In addition to hematopoietic function, another major physiological function of IL-11 is osteogenesis, and osteoblasts constitutively express IL-11 and IL-11Rα. Recent studies have shown that it is an important downstream modulatory factor of TGF-β. In polarized cells, IL-11 secretion leads to cellular dysfunction, and can trigger apoptosis while blocking regeneration. In stromal cells, IL-11 triggers extracellular matrix production and invasion, and the migration of myofibroblasts. IL-11-activated fibroblasts and myofibroblasts secrete cytokines and chemokines, and are strongly proinflammatory. Many basic studies have shown that inhibition of IL-11 protects normal tissues, resists fibrosis, and reduces matrix-driven inflammation.

TSLP and IL-11 share a common mechanistic direction in their inflammatory and fibrotic actions, and studies have found that TSLP can promote fibrosis during the process of tissue repair. If the fibrotic stage continues to progress uninhibited, the outcome of the fibrotic disease will lead to an extensive tissue remodeling and the formation of a permanent scar tissue.

Although developing medicaments targeting IL-11 and TSLP is of great significance, it also faces many challenges, such as pre-clinical evaluation model, low expression, poor stability, complicated process, high variability in quality control and other problems. Therefore, there is an urgent need in the art to develop a multispecific molecule with good multispecificity, good efficacy and easy preparation for better treatment of related diseases.

### SUMMARY OF THE INVENTION

The present application provides a multispecific molecule, comprising a first binding domain capable of targeting IL-11 protein and a second binding domain capable of targeting TSLP protein, the multispecific molecule having one or more of the following properties: (1) being capable of binding a TSLP protein with a KD value of 1×10-⁹M or less, wherein the KD value is determined by surface plasmon resonance; (2) being capable of binding an IL-11 protein with a KD value of 1×10⁻⁹M or less, wherein the KD value is determined by surface plasmon resonance; (3) being capable of blocking the binding of TSLP to a receptor TSLPR/IL7Ra complex; (4) being capable of inhibiting TARC (Thymus and Activation-Regulated Chemokine) production; (5) being able to inhibit cell fibrosis; and (6) blocking the TH2 proinflammatory effect induced by TSLP and IL-11. Moreover, the multispecific molecule provided in the present application has better effect in terms of therapeutic activity, stability or immunogenicity than the IL-11 and/or TSLP antibody present in the prior art.

In one aspect, the present application provides a multispecific molecule comprising a first binding domain capable of targeting IL-11 and a second binding domain capable of targeting TSLP.

In certain embodiments, the first binding domain capable of targeting IL-11 comprises an antibody or an antigen-binding fragment thereof.

In certain embodiments, the antigen-binding fragment is selected from the group consisting of Fab, Fab', F(ab)₂, Fv fragment, F(ab')₂, scFv, di-scFv, VHH and dAb fragment.

In certain embodiments, the antibody is selected from the group consisting of a monoclonal antibody, a single chain antibody, a chimeric antibody, a multispecific antibody, a humanized antibody, a fully human antibody, a heavy chain antibody and/or a nanobody.

In certain embodiments, the first binding domain capable of targeting IL-11 comprises HCDR3, HCDR2 and HCDR1 of the heavy chain variable region VH; wherein the amino acid sequence of the HCDR3 is as set forth in SEQ ID NO: 7, the amino acid sequence of the HCDR2 is as set forth in SEQ ID NO: 6, and the amino acid sequence of the HCDR1 is as set forth in SEQ ID NO: 5.

In certain embodiments, the first binding domain capable of targeting IL-11 comprises an antibody heavy chain variable region VH, and the VH comprises an amino acid sequence as set forth in SEQ ID NO: 4.

In certain embodiments, the first binding domain capable of targeting IL-11 is VHH.

In certain embodiments, the VHH comprises an amino acid sequence as set forth in SEQ ID NO: 4.

In certain embodiments, the first binding domain capable of targeting IL-11 comprises an amino acid sequence as set forth in SEQ ID NO: 4.

In certain embodiments, the first binding domain capable of targeting IL-11 comprises an antibody heavy chain variable region VH and/or an antibody light chain variable region VL.

In certain embodiments, the first binding domain capable of targeting IL-11 can be a sequence known in the prior art.

In certain embodiments, the first binding domain capable of targeting IL-11 can be a sequence cited in CN113056481A.

In certain embodiments, the first binding domain capable of targeting IL-11 comprises HCDR3, HCDR2 and HCDR1 of the heavy chain variable region VH; wherein the HCDR1 can be selected from sequences as set forth in SEQ ID NOs: 64, 65, 95, 104, 110 and 116, the HCDR2 can be selected from sequences as set forth in SEQ ID NOs: 66, 72, 73, 74, 96, 105, 111 and 117, and the HCDR3 can be selected from sequences as set forth in SEQ ID NOs: 67, 68, 97, 106, 112 and 118.

In certain embodiments, the first binding domain capable of targeting IL-11 comprises LCDR3, LCDR2 and LCDR1 of the light chain variable region VL; wherein the LCDR1 can be selected from sequences as set forth in SEQ ID NOs: 69, 70, 71, 98, 101, 107, 113 and 119, the LCDR2 can be selected from sequences as set forth in SEQ ID NOs: 72, 73, 99, 102, 108, 114 and 120, and the LCDR3 can be selected from sequences as set forth in SEQ ID NOs: 74-94, 100, 103, 109, 115 and 121.

In certain embodiments, the first binding domain capable of targeting IL-11 comprises an antibody heavy chain variable region VH, and the VH may comprise an amino acid sequence as set forth in SEQ ID NOs: 122-134.

In certain embodiments, the first binding domain capable of targeting IL-11 comprises an antibody light chain variable region VL, and the VL may comprise an amino acid sequence as set forth in SEQ ID NOs: 135-177.

In certain embodiments, the first binding domain capable of targeting IL-11 comprises an antibody heavy chain constant region.

In certain embodiments, the first binding domain capable of targeting IL-11 comprises an antibody light chain constant region.

In certain embodiments, the second binding domain capable of targeting TSLP comprises an antibody or an antigen-binding fragment thereof.

In certain embodiments, the antigen-binding fragment is selected from the group consisting of Fab, Fab', F(ab)₂, Fv fragment, F(ab')₂, scFv, di-scFv, VHH and dAb.

In certain embodiments, the antibody is selected from the group consisting of a monoclonal antibody, a single chain antibody, a chimeric antibody, a multispecific antibody, a humanized antibody, a fully human antibody, a nanobody, and/or a heavy chain antibody.

In certain embodiments, the second binding domain capable of targeting TSLP comprises HCDR1, HCDR2 and HCDR3 of the heavy chain variable region VH; wherein the amino acid sequence of the HCDR1 is as set forth in SEQ ID NO: 19, the amino acid sequence of the HCDR2 is as set forth in SEQ ID NO: 20, and the amino acid sequence of the HCDR3 is as set forth in SEQ ID NO: 21.

In certain embodiments, the second binding domain capable of targeting TSLP comprises an antibody heavy chain variable region VH, wherein the amino acid sequence of the VH is as set forth in SEQ ID NO: 49, SEQ ID NO: 50, SEQ ID NO: 51, SEQ ID NO: 52 or SEQ ID NO: 53.

In certain embodiments, the second binding domain capable of targeting TSLP comprises LCDR1, LCDR2 and LCDR3 of the light chain variable region VL; wherein the amino acid sequence of the LCDR1 is as set forth in SEQ ID NO: 22, the amino acid sequence of the LCDR2 is as set forth in SEQ ID NO: 23 (GAR), and the amino acid sequence of the LCDR3 is as set forth in SEQ ID NO: 24.

In certain embodiments, the second binding domain capable of targeting TSLP comprises an antibody light chain variable region VL, wherein the amino acid sequence of the VL is as set forth in SEQ ID NO: 54, SEQ ID NO: 55, SEQ ID NO: 56 or SEQ ID NO: 57.

In certain embodiments, the second binding domain capable of targeting TSLP comprises an amino acid sequence selected from any of the following groups of VH and VL:
1) VH: SEQ ID NO: 53, VL: SEQ ID NO: 57;
2) VH: SEQ ID NO: 49, VL: SEQ ID NO: 54;
3) VH: SEQ ID NO: 49, VL: SEQ ID NO: 55;
4) VH: SEQ ID NO: 49, VL: SEQ ID NO: 56;
5) VH: SEQ ID NO: 50, VL: SEQ ID NO: 54;
6) VH: SEQ ID NO: 50, VL: SEQ ID NO: 55;
7) VH: SEQ ID NO: 50, VL: SEQ ID NO: 56;
8) VH: SEQ ID NO: 51, VL: SEQ ID NO: 54;
9) VH: SEQ ID NO: 51, VL: SEQ ID NO: 55;
10) VH: SEQ ID NO: 51, VL: SEQ ID NO: 56;
11) VH: SEQ ID NO: 52, VL: SEQ ID NO: 54;
12) VH: SEQ ID NO: 52, VL: SEQ ID NO: 55; and
13) VH: SEQ ID NO: 52, VL: SEQ ID NO: 56.

In certain embodiments, the second binding domain capable of targeting TSLP comprises an antibody heavy chain constant region.

In certain embodiments, the antibody heavy chain constant region is derived from a human IgG heavy chain constant region.

In certain embodiments, the second binding domain capable of targeting TSLP comprises an antibody light chain constant region.

In certain embodiments, the antibody light chain constant region is derived from a human Igκ constant region.

In certain embodiments, the second binding domain capable of targeting TSLP comprises an antibody heavy chain comprising an amino acid sequence as set forth in SEQ ID NO: 12, SEQ ID NO: 13, SEQ ID NO: 14 and SEQ ID NO: 15.

In certain embodiments, the second binding domain capable of targeting TSLP comprises an antibody light chain comprising an amino acid sequence as set forth in SEQ ID NO: 16, SEQ ID NO: 17 and SEQ ID NO: 18.

In certain embodiments, the second binding domain capable of targeting TSLP can be a sequence known in the prior art.

In certain embodiments, the second binding domain capable of targeting TSLP can be Tezepelumab.

In certain embodiments, the second binding domain capable of targeting TSLP comprises HCDR1, HCDR2 and HCDR3 of the heavy chain variable region VH; wherein the amino acid sequence of the HCDR1 is as set forth in SEQ ID NO: 178, the amino acid sequence of the HCDR2 is as set forth in SEQ ID NO: 179, and the amino acid sequence of the HCDR3 is as set forth in SEQ ID NO: 180.

In certain embodiments, the second binding domain capable of targeting TSLP comprises LCDR3, LCDR2 and LCDR1 of the light chain variable region VL; wherein the amino acid sequence of the LCDR1 is as set forth in SEQ ID NO: 181, the amino acid sequence of the LCDR2 is as set forth in SEQ ID NO: 182, and the amino acid sequence of the LCDR3 is as set forth in SEQ ID NO: 183.

In certain embodiments, the second binding domain capable of targeting TSLP comprises an antibody heavy chain variable region VH, and the VH comprises an amino acid sequence as set forth in SEQ ID NO: 184.

In certain embodiments, the second binding domain capable of targeting TSLP comprises an antibody light chain variable region VL, and the VL comprises an amino acid sequence as set forth in SEQ ID NO: 185.

In certain embodiments, the second binding domain capable of targeting TSLP comprises an antibody heavy chain constant region.

In certain embodiments, the second binding domain capable of targeting TSLP comprises an antibody light chain constant region.

In certain embodiments, the second binding domain capable of targeting TSLP comprises HCDR1, HCDR2 and HCDR3 of the heavy chain variable region; wherein the amino acid sequence of the HCDR1 is as set forth in SEQ ID NO: 186, the amino acid sequence of the HCDR2 is as set forth in SEQ ID NO: 187, and the amino acid sequence of the HCDR3 is as set forth in SEQ ID NO: 188.

In certain embodiments, the second binding domain capable of targeting TSLP comprises HCDR1, HCDR2 and HCDR3 of the heavy chain variable region; wherein the amino acid sequence of the HCDR1 is as set forth in SEQ ID NO: 189, the amino acid sequence of the HCDR2 is as set forth in SEQ ID NO: 190, and the amino acid sequence of the HCDR3 is as set forth in SEQ ID NO: 191.

In certain embodiments, the second binding domain capable of targeting TSLP comprises an antibody heavy chain variable region VH, and the VH comprises an amino acid sequence as set forth in SEQ ID NO: 192.

In certain embodiments, the second binding domain capable of targeting TSLP comprises an antibody heavy chain variable region VH, and the VH comprises an amino acid sequence as set forth in SEQ ID NO: 193.

In certain embodiments, the second binding domain capable of targeting TSLP and the first binding domain capable of targeting IL-11 are directly or indirectly linked.

In certain embodiments, the C-terminus of the first binding domain capable of targeting IL-11 is linked to the N-terminus of the second binding domain capable of targeting TSLP.

In certain embodiments, the C-terminus of the first binding domain capable of targeting IL-11 is linked to the N-terminus of the heavy chain variable region VH of the second binding domain capable of targeting TSLP.

In certain embodiments, the N-terminus of the first binding domain capable of targeting IL-11 is linked to the C-terminus of the second binding domain capable of targeting TSLP.

In certain embodiments, the N-terminus of the first binding domain capable of targeting IL-11 is linked to the C-terminus of the heavy chain constant region of the second binding domain capable of targeting TSLP.

In certain embodiments, the first binding domain capable of targeting IL-11 is linked to the second binding domain capable of targeting TSLP through a linker.

In certain embodiments, the linker comprises a peptide linker.

In certain embodiments, the linker comprises an amino acid sequence of (GGGGS)n, wherein n is any positive integer from 0-10.

In certain embodiments, the linker comprises an amino acid sequence as set forth in GGGGSGGGGS (SEQ ID NO: 3).

In certain embodiments, the multispecific molecule comprises two first binding domains capable of targeting IL-11.

In certain embodiments, the two first binding domains capable of targeting IL-11 are linked directly or indirectly to the N-termini of two heavy chains of the second binding domain capable of targeting TSLP, respectively.

In certain embodiments, the two first binding domains capable of targeting IL-11 are linked directly or indirectly to the C-termini of two heavy chains of the second binding domain capable of targeting TSLP, respectively.

In certain embodiments, the multispecific molecule comprises a first polypeptide chain and a second polypeptide chain.

In certain embodiments, the first polypeptide chain comprises the first binding domain capable of targeting IL-11 and the heavy chain of said second binding domain capable of targeting TSLP.

In certain embodiments, the first polypeptide chain comprises said first binding domain capable of targeting IL-11 and the N-termini of the heavy chain of the second binding domain capable of targeting TSLP linked directly or indirectly.

In certain embodiments, the first polypeptide chain comprises said first binding domain capable of targeting IL-11 and the C-termini of the heavy chain of the second binding domain capable of targeting TSLP linked directly or indirectly.

In certain embodiments, the first polypeptide chain comprises said first binding domain capable of targeting IL-11 and the heavy chain of the second binding domain capable of targeting TSLP linked by a linker.

In certain embodiments, the first polypeptide chain comprises an amino acid sequence as set forth in SEQ ID NO: 58, SEQ ID NO: 59, SEQ ID NO: 60, SEQ ID NO: 61, SEQ ID NO: 62, and SEQ ID NO: 63.

In certain embodiments, the second polypeptide chain comprises a light chain of the second binding domain.

In certain embodiments, the second polypeptide chain comprises an amino acid sequence as set forth in SEQ ID NO: 16, SEQ ID NO: 17 and SEQ ID NO: 18.

In certain embodiments, the multispecific molecule comprises two said first polypeptide chains and two said second polypeptide chains.

In certain embodiments, the first polypeptide chain and the second polypeptide chain are linked by a disulfide bond.

In another aspect, the present application provides a pharmaceutical combination comprising a first antibody capable of targeting IL-11 and a second antibody capable of targeting TSLP.

In certain embodiments, the first antibody and the second antibody are present as a mixture.

In certain embodiments, the first antibody and the second antibody are each present independently.

In certain embodiments, the first antibody and the second antibody can be combined in a certain proportion.

In certain embodiments, the antibody is selected from the group consisting of a monoclonal antibody, a single chain antibody, a chimeric antibody, a multispecific antibody, a humanized antibody, a fully human antibody, a nanobody, and/or a heavy chain antibody.

In certain embodiments, the first antibody comprises HCDR3, HCDR2 and HCDR1 of the heavy chain variable region VH; wherein the amino acid sequence of the HCDR3 is as set forth in SEQ ID NO: 7, the amino acid sequence of the HCDR2 is as set forth in SEQ ID NO: 6, and the amino acid sequence of the HCDR1 is as set forth in SEQ ID NO: 5.

In certain embodiments, the first antibody comprises an antibody heavy chain variable region VH, and the amino acid sequence of the VH is as set forth in SEQ ID NO: 4.

In certain embodiments, the first antibody comprises an antibody heavy chain constant region derived from an IgG heavy chain constant region.

In certain embodiments, the first antibody is a nanobody comprising an amino acid sequence as set forth in SEQ ID NO: 4.

In certain embodiments, the first antibody can be an antibody in the patent WO2023/143556A1.

In certain embodiments, the first antibody capable of targeting IL-11 can be a sequence known in the prior art.

In certain embodiments, the first antibody capable of targeting IL-11 can be a sequence cited in CN113056481A.

In certain embodiments, the first antibody capable of targeting IL-11 comprises HCDR3, HCDR2 and HCDR1 of the heavy chain variable region VH; wherein the HCDR1 can be selected from sequences as set forth in SEQ ID NOs: 64, 65, 95, 104, 110 and 116, the HCDR2 can be selected from sequences as set forth in SEQ ID NOs: 66, 72, 73, 74, 96, 105, 111 and 117, and the HCDR3 can be selected from sequences as set forth in SEQ ID NOs: 67, 68, 97, 106, 112 and 118.

In certain embodiments, the first antibody capable of targeting IL-11 comprises LCDR3, LCDR2 and LCDR1 of the light chain variable region VL; wherein the LCDR1 can be selected from sequences as set forth in SEQ ID NOs: 69, 70, 71, 98, 101, 107, 113 and 119, the LCDR2 can be selected from sequences as set forth in SEQ ID NOs: 72, 73, 99, 102, 108, 114 and 120, and the LCDR3 can be selected from sequences as set forth in SEQ ID NOs: 74-94, 100, 103, 109, 115 and 121.

In certain embodiments, the first antibody capable of targeting IL-11 comprises an antibody heavy chain variable region VH, and the VH may comprise an amino acid sequence as set forth in SEQ ID NOs: 122-134.

In certain embodiments, the first antibody capable of targeting IL-11 comprises an antibody light chain variable region VL, and the VL may comprise an amino acid sequence as set forth in SEQ ID NOs: 135-177.

In certain embodiments, the first antibody capable of targeting IL-11 comprises an antibody heavy chain constant region.

In certain embodiments, the first antibody capable of targeting IL-11 comprises an antibody light chain constant region.

In certain embodiments, the second antibody comprises HCDR3, HCDR2 and HCDR1 of the heavy chain variable region VH; wherein the amino acid sequence of the HCDR3 is as set forth in SEQ ID NO: 21, the amino acid sequence of the HCDR2 is as set forth in SEQ ID NO: 20, and the amino acid sequence of the HCDR1 is as set forth in SEQ ID NO: 19.

In certain embodiments, the second antibody comprises an antibody heavy chain variable region VH, wherein the amino acid sequence of the VH is optionally selected from SEQ ID NO: 49, SEQ ID NO: 50, SEQ ID NO: 51, SEQ ID NO: 52, and SEQ ID NO: 53.

In certain embodiments, the second antibody comprises LCDR1, LCDR2 and LCDR3 of the light chain variable region VL; wherein the amino acid sequence of the LCDR1 is as set forth in SEQ ID NO: 22, the amino acid sequence of LCDR2 is as set forth in SEQ ID NO: 23 (GAR), and the amino acid sequence of LCDR3 is as set forth in SEQ ID NO: 24.

In certain embodiments, the second antibody comprises an antibody light chain variable region VL, wherein the amino acid sequence of the VL is optionally selected from SEQ ID NO: 54, SEQ ID NO: 55, SEQ ID NO: 56, and SEQ ID NO: 57.

In certain embodiments, the second antibody comprises an amino acid sequence selected from any of the following groups of VH and VL:
1) VH: SEQ ID NO: 53, VL: SEQ ID NO: 57;
2) VH: SEQ ID NO: 49, VL: SEQ ID NO: 54;
3) VH: SEQ ID NO: 49, VL: SEQ ID NO: 55;
4) VH: SEQ ID NO: 49, VL: SEQ ID NO: 56;
5) VH: SEQ ID NO: 50, VL: SEQ ID NO: 54;
6) VH: SEQ ID NO: 50, VL: SEQ ID NO: 55;
7) VH: SEQ ID NO: 50, VL: SEQ ID NO: 56;
8) VH: SEQ ID NO: 51, VL: SEQ ID NO: 54;
9) VH: SEQ ID NO: 51, VL: SEQ ID NO: 55;
10) VH: SEQ ID NO: 51, VL: SEQ ID NO: 56;
11) VH: SEQ ID NO: 52, VL: SEQ ID NO: 54;
12) VH: SEQ ID NO: 52, VL: SEQ ID NO: 55; and
13) VH: SEQ ID NO: 52, VL: SEQ ID NO: 56.

In certain embodiments, the second antibody can be an antibody known in the prior art.

In certain embodiments, the second antibody is Tezepelumab.

In certain embodiments, the second antibody capable of targeting TSLP comprises HCDR1, HCDR2 and HCDR3 of the heavy chain variable region VH; wherein the amino acid sequence of the HCDR1 is as set forth in SEQ ID NO: 178, the amino acid sequence of the HCDR2 is as set forth in SEQ ID NO: 179, and the amino acid sequence of the HCDR3 is as set forth in SEQ ID NO: 180.

In certain embodiments, the second antibody capable of targeting TSLP comprises LCDR3, LCDR2 and LCDR1 of the light chain variable region VL; wherein the amino acid sequence of the LCDR1 is as set forth in SEQ ID NO: 181, the amino acid sequence of the LCDR2 is as set forth in SEQ ID NO: 182, and the amino acid sequence of the LCDR3 is as set forth in SEQ ID NO: 183.

In certain embodiments, the second antibody capable of targeting TSLP comprises an antibody heavy chain variable region VH, and the VH comprises an amino acid sequence as set forth in SEQ ID NO: 184.

In certain embodiments, the second antibody capable of targeting TSLP comprises an antibody light chain variable region VL, and the VL comprises an amino acid sequence as set forth in SEQ ID NO: 185.

In certain embodiments, the second antibody capable of targeting TSLP comprises an antibody heavy chain constant region.

In certain embodiments, the second antibody capable of targeting TSLP comprises an antibody light chain constant region.

In certain embodiments, the second binding domain capable of targeting TSLP comprises HCDR1, HCDR2 and HCDR3 of the heavy chain variable region; wherein the amino acid sequence of the HCDR1 is as set forth in SEQ ID NO: 186, the amino acid sequence of the HCDR2 is as set forth in SEQ ID NO: 187, and the amino acid sequence of the HCDR3 is as set forth in SEQ ID NO: 188.

In certain embodiments, the second binding domain capable of targeting TSLP comprises HCDR1, HCDR2 and HCDR3 of the heavy chain variable region; wherein the amino acid sequence of the HCDR1 is as set forth in SEQ ID NO: 189, the amino acid sequence of the HCDR2 is as set forth in SEQ ID NO: 190, and the amino acid sequence of the HCDR3 is as set forth in SEQ ID NO: 191.

In certain embodiments, the second binding domain capable of targeting TSLP comprises an antibody heavy chain variable region VH, and the VH comprises an amino acid sequence as set forth in SEQ ID NO: 192.

In certain embodiments, the second binding domain capable of targeting TSLP comprises an antibody heavy chain variable region VH, and the VH comprises an amino acid sequence as set forth in SEQ ID NO: 193.

In another aspect, the present application also provides one or more isolated nucleic acid moleculeencoding the multispecific molecule.

In another aspect, the present application also provides a vector comprising the nucleic acid molecule.

In another aspect, the present application also provides a cell comprising the nucleic acid molecule or the vector.

In another aspect, the present application also provides a method for preparing the multispecific molecule, comprising culturing the cell under the conditions that enable the expression of the multispecific molecule.

In another aspect, the present application also provides a pharmaceutical composition, comprising the multispecific molecule, the pharmaceutical combination, the nucleic acid molecule, the vector and/or the cell, and optionally a pharmaceutically acceptable adjuvant.

In another aspect, the present application also provides the use of the multispecific molecule, the pharmaceutical combination, the nucleic acid molecule, the vector, the cell and/or the pharmaceutical composition in preparing a medicament for preventing, alleviating and/or treating a disease or disorder

In certain embodiments, the disease and/or disorder includes an IL-11 related disease.

In certain embodiments, the disease and/or disorder includes a TSLP related disease.

In certain embodiments, the disease and/or disorder includes a fibrotic disease, an inflammatory disease or a tumor.

In another aspect, the present application also provides a method for blocking the binding of TSLP to a receptor TSLPR/IL7Ra complex, comprising administering the multispecific molecule and/or the pharmaceutical combination.

In another aspect, the present application also provides a method for inhibiting TARC (Thymus and Activation-Regulated Chemokine) production, comprising administering the multispecific antibody and/or the pharmaceutical combination.

In another aspect, the present application also provides the use of the multispecific molecule, the pharmaceutical combination, the nucleic acid molecule, the vector, the cell and/or the pharmaceutical composition in preparing a medicament for preventing, alleviating and/or treating a disease or disorder.

In certain embodiments, the disease and/or disorder includes an IL-11 related disease.

In certain embodiments, the disease and/or disorder includes a TSLP related disease.

In certain embodiments, the disease and/or disorder includes a fibrotic disease, an inflammatory disease or a tumor.

In another aspect, the present application provides a method for preventing, alleviating and/or treating a disease or disorder, comprising administering to a patient in need thereof the multispecific molecule, the pharmaceutical combination, the nucleic acid molecule, the vector, the cell and/or the pharmaceutical composition of the present application.

In certain embodiments, in the method, the disease and/or disorder includes an IL-11 related disease.

In certain embodiments, in the method, the disease and/or disorder includes a TSLP related disease.

In certain embodiments, in the method, the disease and/or disorder includes a fibrotic disease, an inflammatory disease or a tumor.

In another aspect, the present application provides the multispecific molecule, the pharmaceutical combination, the nucleic acid molecule, the vector, the cell and/or the pharmaceutical composition for use in preventing, alleviating and/or treating a disease or disorder.

In certain embodiments, the disease and/or disorder includes an IL-11 related disease.

In certain embodiments, the disease and/or disorder includes a TSLP related disease.

In certain embodiments, the disease and/or disorder includes a fibrotic disease, an inflammatory disease or a tumor.

In another aspect, the multispecific molecule, the pharmaceutical combination, the nucleic acid molecule, the vector, the cell and/or the pharmaceutical composition provided in the present application has better efficacy as compared to the IL-11 and/or TSLP antibody.

In certain embodiments, the IL-11 antibody can be a sequence cited in CN113056481A.

In certain embodiments, the IL-11 antibody comprises HCDR3, HCDR2 and HCDR1 of the heavy chain variable region VH; wherein the HCDR1 can be selected from sequences as set forth in SEQ ID NOs: 64, 65, 95, 104, 110 and 116, the HCDR2 can be selected from sequences as set forth in SEQ ID NOs: 66, 72, 73, 74, 96, 105, 111 and 117, and the HCDR3 can be selected from sequences as set forth in SEQ ID NOs: 67, 68, 97, 106, 112 and 118.

In certain embodiments, the IL-11 antibody comprises LCDR3, LCDR2 and LCDR1 of the light chain variable region VL; wherein the LCDR1 can be selected from sequences as set forth in SEQ ID NOs: 69, 70, 71, 98, 101, 107, 113 and 119, the LCDR2 can be selected from sequences as set forth in SEQ ID NOs: 72, 73, 99, 102, 108, 114 and 120, and the LCDR3 can be selected from sequences as set forth in SEQ ID NOs: 74-94, 100, 103, 109, 115 and 121.

In certain embodiments, the IL-11 antibody comprises an antibody heavy chain variable region VH, and the VH may comprise an amino acid sequence as set forth in SEQ ID NOs: 122-134.

In certain embodiments, the IL-11 antibody comprises an antibody light chain variable region VL, and the VL may comprise an amino acid sequence as set forth in SEQ ID NOs: 135-177.

In certain embodiments, the TSLP antibody can be Tezepelumab.

In certain embodiments, the TSLP antibody comprises HCDR1, HCDR2 and HCDR3 of the heavy chain variable region VH; wherein the amino acid sequence of the HCDR1 is as set forth in SEQ ID NO: 178, the amino acid sequence of the HCDR2 is as set forth in SEQ ID NO: 179, and the amino acid sequence of the HCDR3 is as set forth in SEQ ID NO: 180.

In certain embodiments, the TSLP antibody comprises LCDR3, LCDR2 and LCDR1 of the light chain variable region VL; wherein the amino acid sequence of the LCDR1 is as set forth in SEQ ID NO: 181, the amino acid sequence of the LCDR2 is as set forth in SEQ ID NO: 182, and the amino acid sequence of the LCDR3 is as set forth in SEQ ID NO: 183.

In certain embodiments, the TSLP antibody comprises an antibody heavy chain variable region VH, and the VH comprises an amino acid sequence as set forth in SEQ ID NO: 184.

In certain embodiments, the TSLP antibody comprises an antibody light chain variable region VL, and the VL comprises an amino acid sequence as set forth in SEQ ID NO: 185.

In certain embodiments, the TSLP antibody comprises an antibody heavy chain constant region. In certain embodiments, the TSLP antibody comprises an antibody light chain constant region.

In certain embodiments, the second binding domain capable of targeting TSLP comprises HCDR1, HCDR2 and HCDR3 of the heavy chain variable region; wherein the amino acid sequence of the HCDR1 is as set forth in SEQ ID NO: 186, the amino acid sequence of the HCDR2 is as set forth in SEQ ID NO: 187, and the amino acid sequence of the HCDR3 is as set forth in SEQ ID NO: 188.

In certain embodiments, the second binding domain capable of targeting TSLP comprises HCDR1, HCDR2 and HCDR3 of the heavy chain variable region; wherein the amino acid sequence of the HCDR1 is as set forth in SEQ ID NO: 189, the amino acid sequence of the HCDR2 is as set forth in SEQ ID NO: 190, and the amino acid sequence of the HCDR3 is as set forth in SEQ ID NO: 191.

In certain embodiments, the second binding domain capable of targeting TSLP comprises an antibody heavy chain variable region VH, and the VH comprises an amino acid sequence as set forth in SEQ ID NO: 192.

In certain embodiments, the second binding domain capable of targeting TSLP comprises an antibody heavy chain variable region VH, and the VH comprises an amino acid sequence as set forth in SEQ ID NO: 193.

In certain embodiments, the efficacy may be a better affinity for IL-11 and/or TSLP.

In certain embodiments, the efficacy may be an inhibitory effect on IL-11 and/or TSLP.

In certain embodiments, the efficacy may be a better therapeutic efficacy for an IL-11 and/or TSLP related disease.

In certain embodiments, the efficacy may be a fibrosis inhibiting effect.

In certain embodiments, the efficacy may be an effect of inhibiting TARC (Thymus and Activation-Regulated Chemokine) production.

In certain embodiments, the efficacy may be an anti-inflammatory efficacy.

Those skilled in the art can easily discern other aspects and advantages of the present application from the following detailed description. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. A brief description of the drawings is as follows:
Fig. 1A shows the structures of antibodies I700019, I700020, and I700021 according to the present application.
Fig. 1B shows the structures of antibodies I700022, I700023, and I700024 according to the present application.
Fig. 2A shows the reduced and non-reduced SDS-PAGE images of TSLP/IL-11 bispecific antibodies I700019, I700020, and I700021 according to the present application.
Fig. 2B shows the reduced and non-reduced SDS-PAGE images of TSLP/IL-11 bispecific antibodies I700022, I700023, and I700024 according to the present application.
Fig. 3 shows the results of binding between the TSLP antibody according to the present application and BaF3-huTSLP-3G11 cells as detected by flow cytometry.
Fig. 4 shows the results of the TSLP antibody according to the present application blocking the binding of TSLP to the receptor TSLPR/IL7Ra complex.
Fig. 5 shows the results of the TSLP antibody according to the present application blocking H_TSLP reporter cell line cell activation caused by TSLP.
Fig. 6 shows that the IL-11 antibody according to the present application blocks IL-11-mediated activation of hIL11 Effector Reporter Cell.
Fig. 7 shows the result of the human TSLP antibody according to the present application blocking the production of TARC by PBMC stimulated by TSLP.
Fig. 8 shows the test results of the inhibitory effects of the TSLP/IL-11 bispecific antibody on cell fibrosis activity.
Fig. 9 shows the test results of the TSLP/IL-11 bispecific antibody blocking TSLP and IL-11-induced TH2 pro-inflammatory effects.

### DETAILED DESCRIPTION

The embodiments of the invention of the present application will be described below with specific examples. Those skilled in the art can easily understand other advantages and effects of the invention of the present application from the disclosure of the specification.

### Definition of Terms

In the present application, the term "multispecific molecule" generally refers to an antibody with a variable region that recognizes one or more epitopes on one or more antigens. Multispecific antibodies include, but are not limited to, full-length antibodies, antibodies having two or more VL and VH domains (such as monoclonal antibodies, chimeric antibodies, humanized antibodies and fully human antibodies), antibody fragments (such as Fab, Fab', F(ab)₂, Fv fragment, F(ab')₂, scFv, di-scFv, VHH and dAb), diabodies, bispecific diabodies and triabodies, antibody fragments that have been covalently or non-covalently linked. In certain embodiments, a multispecific antibody may be a "bispecific antibody" that recognizes two different epitopes on the same or different antigens.

In the present application, the term "IL-11" generally refers to a stromal cell-derived cytokine that belongs to the IL-6 superfamily. The term "IL-11" covers "full-length", unprocessed IL-11, and any form of IL-11 produced by cell processing. In the present application, the term "IL-11" includes mutants, fragments, variants, isoforms and homologues thereof.

In the present application, the term "TSLP", also called "Thymic stromal lymphopoietin", is an interleukin-7 (IL-7)-like cytokine, a mature hTSLP is composed of 131 amino acid residues, and has a typical 4-stranded α-helix bundle structure. The "TSLP" covers "full-length", unprocessed TSLP, and any form of TSLP produced by cell processing. In the present application, the term "TSLP" includes full-length, wild-type TSLP and mutants, fragments, variants, isoforms and homologues thereof.

In the present application, the term "pharmaceutical combination" typically refers to a combination that includes at least two active ingredients/therapeutic agents. In certain embodiments, the active ingredients/therapeutic agents may be prepared as separate preparations (solid, liquid, gel, etc.). In certain embodiments, the active ingredients/therapeutic agents may be contained in different containers, and may also be prepared simultaneously or separately with suitable carriers into desired preparations when required. In certain embodiments, the active ingredients/therapeutic agents may be of different sources. In certain embodiments, the active ingredients/therapeutic agents may exist in a mixture form.

In the present application, the term "isolated" typically refers to artificial collection from a natural state. If an "isolated" substance or component occurs in nature, it may be that the natural environment in which the substance or component is has changed, or that the substance has been isolated from the natural environment, or both. For example, a certain unisolated polynucleotide or polypeptide naturally exists in a living animal, and the same polynucleotide or polypeptide with high purity isolated from the natural state is called "isolated". The term "isolated" does not exclude the presence of artificial or synthetic substances, nor other impure substances that do not affect the activity of the substances.

In the present application, the term "isolated antigen-binding protein" typically refers to a protein which is collected from a natural state by an artificial means and has an antigen-binding ability. The "isolated antigen-binding protein" may include an antigen-binding moiety, and optionally, a framework or frame moiety that allows the antigen-binding moiety to use a conformation that promotes the antigen-binding moiety to bind to an antigen. The antigen-binding protein may comprise, for example, antibody-derived protein framework regions (FRs) or alternative protein framework regions or artificial framework regions with grafted CDRs or CDR derivatives. Such frameworks include, but are not limited to, antibody-derived framework regions including mutations introduced, for example, to stabilize the three-dimensional structure of an antigen-binding protein, as well as fully synthetic framework regions including, for example, biocompatible polymers. Examples of antigen-binding proteins include, but are not limited to: human antibodies and humanized antibodies; chimeric antibodies; recombinant antibodies; single-chain antibodies; bifunctional antibodies; trifunctional antibodies; tetrafunctional antibodies; Fab, Fab', Fv fragments, F(ab')₂, F(ab)₂, scFv, di-scFv and dAb; IgD antibodies; IgE antibodies; IgM antibodies; IgG1 antibodies; IgG2 antibodies; IgG3 antibodies; or IgG4 antibodies and fragments thereof.

In the present application, the term "CDR", also known as a "complementarity determining region", typically refers to a region in an antibody variable domain, the sequence of which is highly variable and/or forms a structure defining loop. Generally, the antibody includes six CDRs; three (HCDR1, HCDR2, HCDR3) in VH and three (LCDR1, LCDR2, LCDR3) in VL. In certain embodiments, a naturally occurring camel antibody consisting only of heavy chains can function normally and stably even in the absence of light chains. An antibody CDR may be determined by a variety of encoding systems, e.g., CCG, Kabat, Chothia, IMGT, and Kabat/Hothia considered comprehensively. These encoding systems are known in the art. For example, the CDRs of the multispecific molecule can be classified according to the IMGT numbering system. For example, the CDRs of the multispecific molecule can be classified according to the Kabat numbering system. For example, the CDRs of the multispecific molecule can be classified according to the Chothia numbering system. For example, in the multispecific molecule, the CDRs of the first binding domain/antibody capable of targeting IL-11 can be classified according to the Kabat numbering system. For example, in the multispecific molecule, the CDRs of the second binding domain/antibody capable of targeting TSLP can be classified according to the IMGT numbering system.

In the present application, the term "FR" typically refers to a more highly conserved moiety of an antibody variable domain, called a framework region. Generally, the variable domains of natural heavy and light chains each includes four FR regions, four (H-FR1, H-FR2, H-FR3, and H-FR4) in VH, and four (L-FR1, L-FR2, L-FR3, and L-FR4) in VL.

In the present application, the terms "variable domain" and "variable region" may be used interchangeably, and typically refer to a moiety of a heavy chain and/or a light chain of an antibody. Variable domains of heavy and light chains can be referred to as "V_{H}" and "V_{L}" (or "VH" and "VL"), respectively. These domains are generally the most variable parts of an antibody (relative to other antibodies of the same type) and comprise antigen-binding sites.

In the present application, the term "VHH" generally refers to an antibody containing a variable antigen-binding domain of a heavy chain antibody. VHH may also be referred to as Nanobody (Nb), heavy chain antibody and/or single domain antibody. For example, the VHH may bind to IL-11. For example, the VHH may be specific for IL-11.

In the present application, the terms "polypeptide molecule", "polypeptide" and "peptide" are used interchangeably and generally refer to polymers of amino acid residues. The term "fusion protein" generally refers to a polypeptide having at least two moieties covalently linked together. Each of these portions can be a polypeptide with different properties. The property may be a biological property, such as in vitro or in vivo activity. The property can also be a simple chemical or physical property, such as binding to a target molecule, catalysis of a reaction. The two portions can be linked directly via a single peptide bond or via a peptide linker.

In the present application, the term "nucleic acid molecule" typically refers to a nucleotide, deoxyribonucleotide or ribonucleotide in an isolated form and of any length, or an analogue isolated from its natural environment or artificially synthesized.

In the present application, the term "vector" generally refers to a nucleic acid carrying tool into which polynucleotide encoding a certain protein can be inserted and the protein is expressed. A vector may be transformed, transduced, or transfected into a host cell to express a genetic material element it carries within the host cell. For example, vectors may include plasmids; phagemids; cosmids; artificial chromosomes such as yeast artificial chromosomes (YAC), bacterial artificial chromosomes (BAC), or P1 derived artificial chromosomes (PAC); phages such as λ phage or M13 phage; animal viruses; and the like. The types of animal viruses used as vectors include retroviruses (including lentiviruses), adenoviruses, adeno-associated viruses, herpes viruses (e.g., herpes simplex virus), poxviruses, baculoviruses, papillomaviruses, and papovaviruses (e.g., SV40). A vector may include a variety of elements that control expression, including a promoter sequence, a transcription initiation sequence, an enhancer sequence, a selection element, and a reporter gene. In addition, the vector may also contain a replication initiation site. The vector may also include components that facilitate to enter cells, such as viral particles, liposomes, or protein coats, but not just these.

In the present application, the term "cell" generally refers to a single cell, cell line, or cell culture that may be, or has been a recipient of a subject plasmid or vector, which includes the nucleic acid molecule according to the present application or the vector according to the present application. The cell can include the progeny of a single cell. Due to natural, accidental or intentional mutations, the progeny may not necessarily be identical to the original mother cell (in the morphology or genome of the total DNA complement). The cell may include a cell transfected in vitro using the vector described in the present application. The cell may be a bacterial cell (e.g., E. coli), yeast cell, or any other eukaryotic cell, e.g., a COS cell, Chinese hamster ovary (CHO) cell, CHO-K1 cell, LNCAP cell, HeLa cell, HEK293 cell, COS-1 cell, or NS0 cell. In certain embodiments, the cell is a mammalian cell. In certain embodiments, the mammalian cell is an HEK293 cell.

In the present application, the term "pharmaceutical composition" typically refers to a composition for use in prevention/treatment of diseases or disorders. The pharmaceutical composition may comprise the isolated antigen-binding protein according to the present application, the nucleic acid molecule according to the present application, the vector according to the present application, and/or the cell according to the present application, and optionally a pharmaceutically acceptable adjuvant. In addition, the pharmaceutical composition may further comprise suitable preparations of one or more (pharmaceutically effective) carriers, stabilizers, excipients, diluents, solubilizers, surfactants, emulsifiers and/or preservatives. Acceptable ingredients of the composition are preferably non-toxic to the recipient at the doses and concentrations used. The pharmaceutical composition of the present application includes, but is not limited to, a liquid, frozen or freeze-dried composition.

In the present application, the term "pharmaceutically acceptable carrier" typically includes a pharmaceutically acceptable carrier, excipient or stabilizer that is non-toxic at the dose and concentration used to a cell or mammal exposed thereto. A physiologically acceptable carrier may include, for example, a buffering agent; antioxidant; low molecular weight (less than about 10 residues) polypeptide; protein; hydrophilic polymer; amino acid; monosaccharide, disaccharide and any other carbohydrate; chelating agent; sugar alcohol; salt forming counter ion, e.g., sodium; and/or non-ionic surfactant.

In the present application, the term "subject" typically refers to a human or a non-human animal, including, but is not limited to a cat, dog, horse, pig, cow, sheep, rabbit, mouse, rat, or monkey.

In the present application, a protein, polypeptide, and/or amino acid sequence involved should also be understood to include at least the following: variants or homologues having the same or similar functions as the protein or polypeptide.

In the present application, the variants may be proteins or polypeptides with substitution, deletion or addition of one or more amino acids in the amino acid sequence of the protein and/or the polypeptide (e.g., an antibody or fragment thereof specifically binding to a CD73 protein). For example, the functional variants may include proteins or polypeptides with amino acid alterations through substitution, deletion and/or insertion of at least 1, e.g., 1-30, 1-20, 1-10, 1, 2, 3, 4 or 5 amino acids. The functional variant may substantially maintain the biological properties of the protein or the polypeptide prior to change (e.g., substitution, deletion, or addition). For example, the functional variant may retain at least 60%, 70%, 80%, 90% or 100% of the biological activity (e.g., antigen-binding ability) of the protein or the polypeptide prior to the alteration. For example, the substitution may be conservative substitution.

In the present application, the homologues may be proteins or polypeptides having at least about 85% (e.g., having at least about 85%, about 90%, about 91%, about 92%, about 93%, about 94%, about 95%, about 96%, about 97%, about 98%, about 99% or higher) sequence homology with the amino acid sequence of the protein and/or the polypeptide (e.g., an antibody or fragment thereof specifically binding to a CD73 protein).

In the present application, the homology typically refers to similarity, likeness or association between two or more sequences. The "percentage of sequence homology" can be calculated by comparing two sequences to be aligned in a comparison window, determining the number of sites in which the same nucleic acid base (e.g., A, T, C, G, and I) or the same amino acid residue (e.g., Ala, Pro, Ser, Thr, Gly, Val, Leu, Ile, Phe, Tyr, Trp, Lys, Arg, His, Asp, Glu, Asn, Gln, Cys and Met) are present in the two sequences to obtain the number of matching sites, dividing the number of matching sites by the total number of sites in the comparison window (i.e., window size), and multiplying the result by 100 to produce the percentage of sequence homology. Alignment for the purpose of determining percent sequence homology can be accomplished in a variety of ways known in the art.

In the present application, the term "include" typically refers to the meaning of include, comprise, contain, or encompass. In some cases, it also means "be" and "consist of".

In the present application, the term "about" typically refers to a change in a range of 0.5-10% greater or less than a specified value, e.g., a change in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% greater or less than the specified value.

### DETAILED DESCRIPTION of THE PRESENT INVENTION

### IL-11 antibody/isolated IL-11 antigen-binding protein

In an aspect, the present application provides an IL-11 antibody/isolated IL-11 antigen-binding protein.

In the present application, the IL-11 antibody/isolated IL-11 antigen-binding protein may comprise at least one CDR of an antibody heavy chain variable region VH, and the amino acid sequence of the VH is as set forth in SEQ ID NO: 4.

In the present application, the IL-11 antibody/isolated IL-11 antigen-binding protein may comprise HCDR3, and the amino acid sequence of the HCDR3 is as set forth in SEQ ID NO: 7.

In the present application, the IL-11 antibody/isolated IL-11 antigen-binding protein may comprise HCDR2, and the amino acid sequence of the HCDR2 is as set forth in SEQ ID NO: 6.

In the present application, the IL-11 antibody/isolated IL-11 antigen-binding protein may comprise HCDR1, and the amino acid sequence of the HCDR1 is as set forth in SEQ ID NO: 5.

In the present application, the IL-11 antibody/isolated IL-11 antigen-binding protein may include HCDR3, HCDR2 and HCDR1, the amino acid sequence of the HCDR3 is as set forth in SEQ ID NO: 7, the amino acid sequence of the HCDR2 is as set forth in SEQ ID NO: 6, and the amino acid sequence of the HCDR1 is as set forth in SEQ ID NO: 5.

In the present application, the IL-11 antibody/isolated IL-11 antigen-binding protein may comprise at least one CDR in the VH according to the present application. The CDR can be obtained by classification according to any classification method.

For example, the CDRs of the IL-11 antibody/isolated IL-11 antigen-binding protein can be classified according to Kabat.

In the present application, the antibody framework region FR refers to the portion of the antibody variable region that exists between the more divergent (i.e., hypervariable) CDRs. Such framework regions are typically referred to as frameworks 1 to 4 (FR1, FR2, FR3, and FR4) and provide a scaffold for presenting the six CDRs (three from the heavy chain and three from the light chain) in three dimensional space to form an antigen binding surface.

In the present application, the IL-11 antibody/isolated IL-11 antigen-binding protein may comprise H-FR1, the C-terminus of the H-FR1 is directly or indirectly linked to the N-terminus of the HCDR1, and the amino acid sequence of the H-FR1 is as set forth in SEQ ID NO: 8.

In the present application, the IL-11 antibody/isolated IL-11 antigen-binding protein may comprise H-FR2, the H-FR2 is located between the HCDR1 and the HCDR2, and the amino acid sequence of the H-FR2 is as set forth in SEQ ID NO: 9.

In the present application, the IL-11 antibody/isolated IL-11 antigen-binding protein may comprise H-FR3, the H-FR3 is located between the HCDR2 and the HCDR3, and the amino acid sequence of the H-FR3 is as set forth in SEQ ID NO: 10.

In the present application, the IL-11 antibody/isolated IL-11 antigen-binding protein may comprise H-FR4, the N-terminus of the H-FR4 is linked to the C-terminus of the HCDR3, and the amino acid sequence of the H-FR4 is as set forth in SEQ ID NO: 11.

In the present application, the IL-11 antibody/isolated IL-11 antigen-binding protein may comprise a variable region VH, and the amino acid sequence of the VH is as set forth in SEQ ID NO: 4.

In the present application, the IL-11 antibody/isolated IL-11 antigen-binding protein may include an antibody or an antigen-binding fragment thereof.

For example, the antigen-binding fragment includes Fab, Fab', F(ab)₂, Fv fragment, F(ab')₂, scFv, di-scFv, VHH and/or dAb.

For example, the antibody is selected from the group consisting of a monoclonal antibody, a chimeric antibody, a humanized antibody, a fully human antibody, a nanobody and/or a heavy chain antibody.

In the present application, the IL-11 antibody/isolated IL-11 antigen-binding protein may comprise VHH, and the amino acid sequence of the VHH is as set forth in SEQ ID NO: 4.

In the present application, the IL-11 antibody/isolated IL-11 antigen-binding protein may comprise an antibody heavy chain constant region. The antibody heavy chain constant region may be derived from a human IgG heavy chain constant region. In certain embodiments, the isolated antigen-binding protein may include an antibody heavy chain constant region which can be derived from a human IgG1 heavy chain constant region. In certain embodiments, the isolated antigen-binding protein may include an antibody heavy chain constant region which can be derived from a human IgG heavy chain constant region and undergo amino acid mutation.

In the present application, the IL-11 antibody/isolated IL-11 antigen-binding protein may be a sequence known in the prior art.

In the present application, the IL-11 antibody/isolated IL-11 antigen-binding protein may be the sequence recorded in CN113056481A.

In the present application, the IL-11 antibody/isolated IL-11 antigen-binding protein may comprise HCDR3, HCDR2 and HCDR1 of the heavy chain variable region VH; wherein the HCDR1 can be selected from sequences as set forth in SEQ ID NOs: 64, 65, 95, 104, 110 and 116, the HCDR2 can be selected from sequences as set forth in SEQ ID NOs: 66, 72, 73, 74, 96, 105, 111 and 117, and the HCDR3 can be selected from sequences as set forth in SEQ ID NOs: 67, 68, 97, 106, 112 and 118.

In the present application, the IL-11 antibody/isolated IL-11 antigen-binding protein may comprise LCDR3, LCDR2 and LCDR1 of the light chain variable region VL; wherein the LCDR1 can be selected from sequences as set forth in SEQ ID NOs: 69, 70, 71, 98, 101, 107, 113 and 119, the LCDR2 can be selected from sequences as set forth in SEQ ID NOs: 72, 73, 99, 102, 108, 114 and 120, and the LCDR3 can be selected from sequences as set forth in SEQ ID NOs: 74-94, 100, 103, 109, 115 and 121.

In the present application, the IL-11 antibody/isolated IL-11 antigen-binding protein may comprise an antibody heavy chain variable region VH, and the VH may comprise amino acid sequences as set forth in SEQ ID NOs: 122-134.

In the present application, the IL-11 antibody/isolated IL-11 antigen-binding protein may comprise an antibody light chain variable region VL, and the VL may comprise amino acid sequences as set forth in SEQ ID NOs: 135-177.

In the present application, the IL-11 antibody/isolated IL-11 antigen-binding protein comprises an antibody heavy chain constant region.

In the present application, the IL-11 antibody/isolated IL-11 antigen-binding protein comprises an antibody light chain constant region.

In addition, it is to be noted that the isolated antigen-binding protein according to the present application may include a heavy chain and/or light chain sequence having one or more conserved sequence modifications present in the antigen-binding protein. The "conserved sequence modifications" refer to amino acid modifications that do not significantly affect or change the antibody binding properties. Such conserved modifications include amino acid substitutions, additions, and deletions. The modification can be introduced into the isolated antigen-binding proteins according to the present application by standard techniques known in the art, such as point mutations and PCR-mediated mutations. The conserved amino acid substitution refers to the substitution of amino acid residues with amino acid residues having similar side chains. The group of amino acid residues having similar side chains is known in the art. These groups of amino acid residues include those with basic side chains (e.g., lysine, arginine, and histidine), acidic side chains (e.g., aspartic acid, and glutamic acid), side chains without polarity (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine) and aromatic side chains (e.g., tyrosine, phenylalanine, tryptophan, and histidine). In certain embodiments, one or more amino acid residues in the CDR region of the isolated antigen-binding protein according to the present application may be substituted with other amino acid residues of the same side chain group. Those skilled in the art know that some conserved sequence modifications do not cause loss of antigen binding activity.

### TSLP antibody/isolated TSLP antigen-binding protein

In another aspect, the present application provides a TSLP antibody/isolated TSLP antigen-binding protein.

In the present application, the TSLP antibody/isolated TSLP antigen-binding protein comprises HCDR3, and the amino acid sequence of the HCDR3 is as set forth in SEQ ID NO: 21.

In the present application, the TSLP antibody/isolated TSLP antigen-binding protein may further comprise HCDR2, and the amino acid sequence of the HCDR2 is as set forth in SEQ ID NO: 20.

In the present application, the TSLP antibody/isolated TSLP antigen-binding protein may further comprise HCDR1, and the amino acid sequence of the HCDR1 is as set forth in SEQ ID NO: 19.

In the present application, the TSLP antibody/isolated TSLP antigen-binding protein may comprise HCDR3, HCDR2 and HCDR1. For example, the amino acid sequence of the isolated antigen-binding protein HCDR3 is as forth in SEQ ID NO: 21, that of the HCDR2 is as forth in SEQ ID NO: 20, and that of the HCDR1 is as forth in SEQ ID NO: 19.

In the present application, the TSLP antibody/isolated TSLP antigen-binding protein may comprise LCDR3, and the amino acid sequence of the LCDR3 is as set forth in SEQ ID NO: 24.

In the present application, the TSLP antibody/isolated TSLP antigen-binding protein may further comprise LCDR2, and the amino acid sequence of the LCDR2 is as set forth in SEQ ID NO: 23 (GAR).

In the present application, the TSLP antibody/isolated TSLP antigen-binding protein may further comprise LCDR1, and the amino acid sequence of the LCDR1 is as set forth in SEQ ID NO: 22.

In the present application, the TSLP antibody/isolated TSLP antigen-binding protein may comprise LCDR3, LCDR2 and LCDR1. For example, the amino acid sequence of the LCDR3 of the isolated antigen-binding protein is as forth in SEQ ID NO: 24, that of the LCDR2 is as forth in SEQ ID NO: 23 (GAR), and that of the LCDR1 is as forth in SEQ ID NO: 22.

In the present application, the TSLP antibody/isolated TSLP antigen-binding protein may comprise HCDR3, HCDR2, HCDR1, LCDR3, LCDR2 and LCDR1. For example, for the isolated antigen-binding protein according to the present application, the amino acid sequence of the HCDR3 is as set forth in SEQ ID NO: 21, that of the HCDR2 is as set forth in SEQ ID NO: 20, that of the HCDR1 is as set forth in SEQ ID NO: 19, that of the LCDR3 is as set forth in SEQ ID NO: 24, that of the LCDR2 is as set forth in SEQ ID NO: 23 (GAR), and that of the LCDR1 is as set forth in SEQ ID NO: 22.

In the present application, the TSLP antibody/isolated TSLP antigen-binding protein may comprise H-FR1, the C-terminus of the H-FR1 may be directly or indirectly linked to the N-terminus of the HCDR1, and the amino acid sequence of the H-FR1 may be as set forth in SEQ ID NO: 25, SEQ ID NO: 26 and SEQ ID NO: 27.

In the present application, the TSLP antibody/isolated TSLP antigen-binding protein may comprise H-FR2, the H-FR2 may be located between the HCDR1 and the HCDR2, and the amino acid sequence of the H-FR2 may be as set forth in SEQ ID NO: 28, SEQ ID NO: 29, SEQ ID NO: 30 and SEQ ID NO: 31.

In the present application, the TSLP antibody/isolated TSLP antigen-binding protein may comprise H-FR3, the H-FR3 may be located between the HCDR2 and the HCDR3, and the amino acid sequence of the H-FR3 may be as set forth in SEQ ID NO: 32, SEQ ID NO: 33, SEQ ID NO: 34, SEQ ID NO: 35 and SEQ ID NO: 36.

In the present application, the TSLP antibody/isolated TSLP antigen-binding protein may comprise H-FR4, the N-terminus of the H-FR4 may be linked to the C-terminus of the HCDR3, and the amino acid sequence of the H-FR4 may be as set forth in SEQ ID NO: 37 and SEQ ID NO: 38.

In the present application, the TSLP antibody/isolated TSLP antigen-binding protein may comprise L-FR1, the C-terminus of the L-FR1 may be directly or indirectly linked to the N-terminus of the LCDR1, and the amino acid sequence of the L-FR1 may be as set forth in SEQ ID NO: 39 and SEQ ID NO: 40.

In the present application, the TSLP antibody/isolated TSLP antigen-binding protein may comprise L-FR2, the L-FR2 may be located between the LCDR1 and the LCDR2, and the amino acid sequence of the L-FR2 may be as set forth in SEQ ID NO: 41, SEQ ID NO: 42 and SEQ ID NO: 43.

In the present application, the TSLP antibody/isolated TSLP antigen-binding protein may comprise L-FR3, the L-FR3 may be located between the LCDR2 and the LCDR3, and the amino acid sequence of the L-FR3 may be as set forth in SEQ ID NO: 44, SEQ ID NO: 45, SEQ ID NO: 46 and SEQ ID NO: 47.

In the present application, the TSLP antibody/isolated TSLP antigen-binding protein may comprise L-FR4, the N-terminus of the L-FR4 may be linked to the C-terminus of the LCDR3, and the amino acid sequence of the L-FR4 may be as set forth in SEQ ID NO: 48.

In the present application, the TSLP antibody/isolated TSLP antigen-binding protein may comprise VH, and the VH may comprise amino acid sequences as set forth in SEQ ID NO: 49, SEQ ID NO: 50, SEQ ID NO: 51, SEQ ID NO: 52 and SEQ ID NO: 53.

In the present application, the TSLP antibody/isolated TSLP antigen-binding protein may comprise VL, and the VL may comprise amino acid sequences as set forth in SEQ ID NO: 54, SEQ ID NO: 55, SEQ ID NO: 56 and SEQ ID NO: 57.

In the present application, the TSLP antibody/isolated TSLP antigen-binding protein may comprise the VH and VL.

For example, the VH may comprise an amino acid sequence as set forth in SEQ ID NO: 53, and the VL may comprise an amino acid sequence as set forth in SEQ ID NO: 57.

For example, the VH may comprise an amino acid sequence as set forth in SEQ ID NO: 49, and the VL may comprise an amino acid sequence as set forth in SEQ ID NO: 54.

For example, the VH may comprise an amino acid sequence as set forth in SEQ ID NO: 49, and the VL may comprise an amino acid sequence as set forth in SEQ ID NO: 55.

For example, the VH may comprise an amino acid sequence as set forth in SEQ ID NO: 49, and the VL may comprise an amino acid sequence as set forth in SEQ ID NO: 56.

For example, the VH may comprise an amino acid sequence as set forth in SEQ ID NO: 50, and the VL may comprise an amino acid sequence as set forth in SEQ ID NO: 54.

For example, the VH may comprise an amino acid sequence as set forth in SEQ ID NO: 50, and the VL may comprise an amino acid sequence as set forth in SEQ ID NO: 55.

For example, the VH may comprise an amino acid sequence as set forth in SEQ ID NO: 50, and the VL may comprise an amino acid sequence as set forth in SEQ ID NO: 56.

For example, the VH may comprise an amino acid sequence as set forth in SEQ ID NO: 51, and the VL may comprise an amino acid sequence as set forth in SEQ ID NO: 54.

For example, the VH may comprise an amino acid sequence as set forth in SEQ ID NO: 51, and the VL may comprise an amino acid sequence as set forth in SEQ ID NO: 55.

For example, the VH may comprise an amino acid sequence as set forth in SEQ ID NO: 51, and the VL may comprise an amino acid sequence as set forth in SEQ ID NO: 56.

For example, the VH may comprise an amino acid sequence as set forth in SEQ ID NO: 52, and the VL may comprise an amino acid sequence as set forth in SEQ ID NO: 54.

For example, the VH may comprise an amino acid sequence as set forth in SEQ ID NO: 52, and the VL may comprise an amino acid sequence as set forth in SEQ ID NO: 55.

For example, the VH may comprise an amino acid sequence as set forth in SEQ ID NO: 52, and the VL may comprise an amino acid sequence as set forth in SEQ ID NO: 56.

In the present application, the TSLP antibody/isolated TSLP antigen-binding protein may comprise at least one CDR in the VH according to the present application. In the present application, the isolated antigen-binding protein may comprise at least one CDR in the VL according to the present application. The CDR can be obtained by classification according to any classification method.

For example, the CDRs of the TSLP antibody/isolated TSLP antigen-binding protein can be classified according to IMGT.

In the present application, the TSLP antibody/isolated TSLP antigen-binding protein may comprise the HCDR1, HCDR2 and HCDR3 in the VH according to the present application. The VH may comprise an amino acid sequence selected from any one of SEQ ID NO: 49, SEQ ID NO: 50, SEQ ID NO: 51, SEQ ID NO: 52 and SEQ ID NO: 53.

In the present application, the TSLP antibody/isolated TSLP antigen-binding protein may comprise the LCDR1, LCDR2 and LCDR3 in the VL according to the present application. The VL may comprise an amino acid sequence selected from any one of SEQ ID NO: 54, SEQ ID NO: 55, SEQ ID NO: 56 and SEQ ID NO: 57.

In the present application, the TSLP antibody/isolated TSLP antigen-binding protein may comprise an antibody heavy chain constant region. The antibody heavy chain constant region may be derived from a human IgG heavy chain constant region. In certain embodiments, the isolated antigen-binding protein may include an antibody heavy chain constant region which can be derived from a human IgG1 heavy chain constant region.

In the present application, the TSLP antibody/isolated TSLP antigen-binding protein may comprise an antibody light chain constant region. The antibody light chain constant region may be derived from a human Igκ constant region.

In certain embodiments, the antigen-binding fragment may include Fab, Fab', Fv fragment, F(ab')₂, F(ab)₂, scFv, di-scFv and/or dAb.

In certain embodiments, the antibody may include a monoclonal antibody, a chimeric antibody, a humanized antibody, a fully human antibody, a nanobody, and/or a heavy chain antibody.

In the present application, the TSLP antibody/isolated TSLP antigen-binding protein may be an antibody known in the prior art.

In the present application, the TSLP antibody/isolated TSLP antigen-binding protein is Tezepelumab.

In the present application, the TSLP antibody/isolated TSLP antigen-binding protein may comprise HCDR1, HCDR2 and HCDR3 of a heavy chain variable region VH; wherein the amino acid sequence of the HCDR1 is as set forth in SEQ ID NO: 178, that of the HCDR2 is as set forth in SEQ ID NO: 179, and that of the HCDR3 is as set forth in SEQ ID NO: 180.

In the present application, the TSLP antibody/isolated TSLP antigen-binding protein may comprise LCDR3, LCDR2 and LCDR1 of a light chain variable region VL; wherein the amino acid sequence of the LCDR1 is as set forth in SEQ ID NO: 181, that of the LCDR2 is as set forth in SEQ ID NO: 182, and that of the LCDR3 is as set forth in SEQ ID NO: 183.

In the present application, the TSLP antibody/isolated TSLP antigen-binding protein may comprise an antibody heavy chain variable region VH, and the VH may comprise an amino acid sequence as set forth in SEQ ID NO: 184.

In the present application, the TSLP antibody/isolated TSLP antigen-binding protein may comprise an antibody light chain variable region VL, and the VL may comprise an amino acid sequence as set forth in SEQ ID NO: 185.

In the present application, the TSLP antibody/isolated TSLP antigen-binding protein may comprise an antibody heavy chain constant region.

In the present application, the TSLP antibody/isolated TSLP antigen-binding protein may comprise an antibody light chain constant region.

In certain embodiments, the second binding domain capable of targeting TSLP comprises HCDR1, HCDR2 and HCDR3 of a heavy chain variable region; wherein the amino acid sequence of the HCDR1 is as set forth in SEQ ID NO: 186, that of the HCDR2 is as set forth in SEQ ID NO: 187, and that of the HCDR3 is as set forth in SEQ ID NO: 188.

In certain embodiments, the second binding domain capable of targeting TSLP comprises HCDR1, HCDR2 and HCDR3 of a heavy chain variable region; wherein the amino acid sequence of the HCDR1 is as set forth in SEQ ID NO: 189, that of the HCDR2 is as set forth in SEQ ID NO: 190, and that of the HCDR3 is as set forth in SEQ ID NO: 191.

In certain embodiments, the second binding domain capable of targeting TSLP comprises an antibody heavy chain variable region VH, and the VH comprises an amino acid sequence as set forth in SEQ ID NO: 192.

In certain embodiments, the second binding domain capable of targeting TSLP comprises an antibody heavy chain variable region VH, and the VH comprises an amino acid sequence as set forth in SEQ ID NO: 193.

### Multispecific molecules

In another aspect, the present application provides a multispecific molecule comprising a first binding domain capable of binding to IL-11. In the present application, the first binding domain of the multispecific antibody may comprise the IL-11 antibody/isolated IL-11 antigen-binding protein according to the present application.

In the present application, the multispecific antibody may further include a second targeting moiety, which may include a second binding domain capable of binding to TSLP. In the present application, the second binding domain of the multispecific antibody may comprise the TSLP antibody/isolated TSLP antigen-binding protein according to the present application.

In the present application, the first binding domain capable of binding to IL-11 and the second binding domain capable of binding to TSLP include Fab, Fab', Fv fragment, F(ab')₂, scFv, di-scFv and/or dAb. For another example, the first binding domain capable of binding to IL-11 and the second binding domain capable of binding to TSLP are selected from the group consisting of: monoclonal antibodies, chimeric antibodies, humanized antibodies, fully human antibodies, single-chain antibodies, nanobodies and/or heavy chain antibodies.

In the present application, the multispecific molecule may be a bispecific antibody. Bispecific antibody (bsAb) can be a non-natural antibody that can simultaneously target two different antigens or proteins, block one or more signal pathways, and stimulate a specific immune response. Its specificity and bifunctionality play an increasingly important role in tumor immunotherapy and it has become a research hotspot in the field of antibody engineering therapy for tumors in the world today. Studies have shown that bispecific antibodies mainly mediate the killing of tumors by immune cells in tumor immunotherapy; combine dual targets, block signal pathways, and exert unique or overlapping functions to effectively prevent drug resistance; have strong specificity, targeting ability, and reduced off-target toxicity; and effectively reduce treatment costs. Therefore, the use of bispecific antibody drugs can reduce the chance of tumor cell escape, eliminate tumor cells, and improve efficacy. Bispecific antibodies can be prepared by means of dual hybridoma cells, chemical coupling, recombinant gene, etc. Among others, the recombinant gene technology has strong flexibility in binding site and yield. The structures of bispecific antibodies mainly include bispecific antibodies containing Fc fragments (IgG-like bispecific antibodies, which have Fc-mediated effector functions) and bispecific antibodies without Fc fragments (non-IgG-like bispecific antibodies, which exert their effects through antigen binding and have the advantages of small molecular weight and low immunogenicity).

In the present application, in the multispecific molecule, the first binding domain capable of targeting IL-11 and the second binding domain capable of targeting TSLP can be directly or indirectly linked. For example, the C-terminus of the first binding domain capable of targeting IL-11 is linked to the N-terminus of the second binding domain capable of targeting TSLP. For example, the C-terminus of the first binding domain capable of targeting IL-11 is linked to the N-terminus of the heavy chain variable region VH of the second binding domain capable of targeting TSLP. For example, the N-terminus of the first binding domain capable of targeting IL-11 is linked to the C-terminus of the second binding domain capable of targeting TSLP. For example, the N-terminus of the first binding domain capable of targeting IL-11 is linked to the C-terminus of the heavy chain constant region of the second binding domain capable of targeting TSLP. For example, the first binding domain capable of targeting IL-11 is linked to the second binding domain capable of targeting TSLP through a linker. The linker may include a peptide linker. For example, the linker may include an amino acid sequence of (GGGGS)n, where n is any positive integer from 0 to 10. For example, the linker includes an amino acid sequence as set forth in GGGGSGGGGS (SEQ ID NO: 3).

In the present application, the multispecific molecule may comprise two first binding domains capable of targeting IL-11, wherein the two first binding domains capable of targeting IL-11 are linked directly or indirectly to the N-termini of two heavy chains of the second binding domain capable of targeting TSLP, respectively. For example, the two first binding domains capable of targeting IL-11 are linked directly or indirectly to the C-termini of two heavy chains of the second binding domain capable of targeting TSLP, respectively. For example, the two first binding domains capable of targeting IL-11 are linked directly or indirectly to the N-termini of two heavy chains of the second binding domain capable of targeting TSLP, respectively.

In the present application, the multispecific molecule may comprise a first polypeptide chain and a second polypeptide chain, wherein the first polypeptide chain may comprise the first binding domain capable of targeting IL-11 and the heavy chain of the second binding domain capable of targeting TSLP, and the second polypeptide chain may comprise the light chain of the second binding domain. In the present application, the multispecific molecule comprises two said first polypeptide chains and two said second polypeptide chains. For example, the first polypeptide chain comprises the first binding domain capable of targeting IL-11 and the N-termini of the heavy chain of the second binding domain capable of targeting TSLP linked directly or indirectly. For example, the first polypeptide chain comprises the first binding domain capable of targeting IL-11 and the C-termini of the heavy chain of the second binding domain capable of targeting TSLP linked directly or indirectly. For example, the first polypeptide chain comprises the first binding domain capable of targeting IL-11 and the heavy chain of the second binding domain capable of targeting TSLP linked by a linker. For example, the first polypeptide chain may comprise an amino acid sequence selected from any one of SEQ ID NO: 58, SEQ ID NO: 59, SEQ ID NO: 60, SEQ ID NO: 61, SEQ ID NO: 62 and SEQ ID NO: 63, or may be an IL-11 antibody/antigen-binding fragment known in the prior art. For example, the second polypeptide chain may comprise amino acid sequences set forth in SEQ ID NO: 16, SEQ ID NO: 17 and SEQ ID NO: 18, or may be a TSLP antibody/antigen binding fragment known in the prior art.

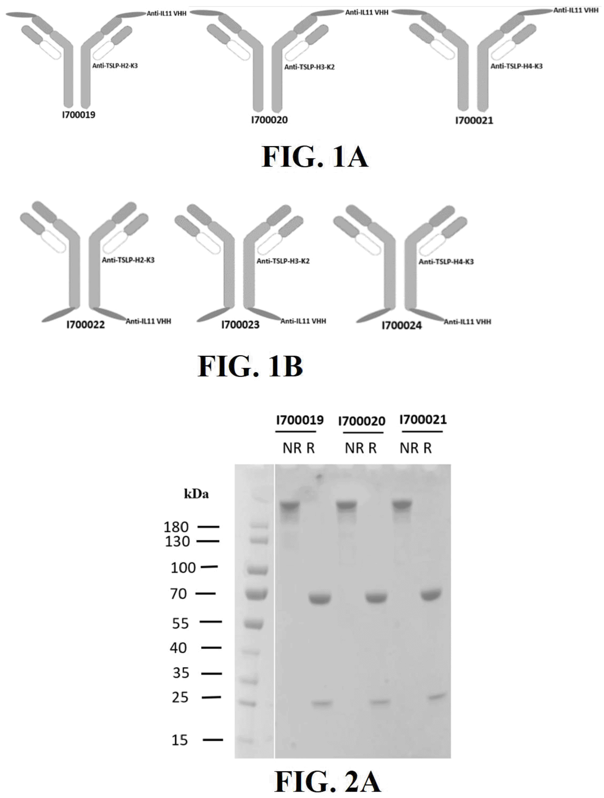

In the present application, the structure of the multispecific molecule may belong to VHH-IgG, that is, the N-termini or C-termini of the two heavy chains of an IgG antibody targeting the TSLP antigen are both linked to the VHH fragment of another nanobody targeting the IL-11 antigen. In the present application, the structure of the multispecific molecule can be a configuration in which VHH is linked to the N-terminus of an IgG antibody, or a configuration in which VHH is linked to the C-terminus of an IgG antibody heavy chain. For example, the structure of the multispecific molecule can be as shown in Fig. 1A and Fig. 1B. For example, the structure of the multispecific molecule can be as set forth in antibodies I700019, I700020, I700021, I700022, I700023, and I700024.

### Pharmaceutical combination

In another aspect, the present application provides a pharmaceutical combination, which comprises a first antibody capable of targeting IL-11 and a second antibody capable of targeting TSLP.

In the present application, the first antibody capable of targeting IL-11 may include the IL-11 antibody/isolated IL-11 antigen-binding protein according to the present application, or may be one or more of the currently known IL-11 antibodies. For example, it is the antibody in patent WO2023/143556A1. For example, it is the antibody in patent CN113056481A.

In the present application, the second antibody capable of targeting TSLP may include the TSLP antibody/isolated TSLP antigen-binding protein according to the present application, or may be one or more of the currently known TSLP antibodies. For example, it is Tezepelumab. For example, it is the antibody in patent CN102782149B.

In the present application, the pharmaceutical combination may refer to a product resulting from the mixing or combination of more than one active ingredients, and includes both fixed and non-fixed combinations of the active ingredients. A fixed combination may mean that the active ingredients (e.g., IL-11 antibodies, TSLP antibodies and multispecific molecules according to the present application) and one or more combinations thereof are all administered to a patient simultaneously in the form of a single entity or dosage. A non-fixed combination may mean that the active ingredient and one or more combinations thereof are administered to a patient as separate entities simultaneously, concurrently or sequentially (with no specific time limits), wherein such administration provides therapeutically effective levels of two compounds in the patient. The latter also applies to cocktail therapy, for example, the administration of three or more active ingredients.

### Isolated nucleic acid molecule(s), vector(s) and cell(s)

In another aspect, the present application further provides isolated nucleic acid molecule(s). The nucleic acid molecule(s) can encode the multispecific molecules according to the present application. For example, each of the nucleic acid molecule(s) can encode the multispecific molecule of full length, or it can encode a part of it (for example, one or more of the first binding domain targeting IL-11, the second binding domain targeting TSLP, IL-11 antibody, TSLP antibody, HCDR1-3, LCDR1-3, VL, VH, light chain or heavy chain).

The nucleic acid molecules according to the present application may be isolated. For example, it may be produced or synthesized by: (i) amplification in vitro, such as amplification by polymerase chain reaction (PCR), (ii) clonal recombination, (iii) purification, such as by enzymatic digestion and gel electrophoresis fractionation, or (iv) synthesis, such as chemical synthesis. In certain embodiments, the isolated nucleic acid is a nucleic acid molecule prepared by recombinant DNA technology.

In the present application, nucleic acids encoding the multispecific antibodies can be prepared by a variety of methods known in the art, including but not limited to, overlap extension PCR using restricted fragment manipulation or using synthetic oligonucleotides. For specific operations, see Sambrook et al., Molecular Cloning, A Laboratory Manual, Cold Spring Harbor Laboratory Press, Cold Spring Harbor, N.Y., 1989; and Ausube et al. Current Protocols in Molecular Biology, Greene Publishing and Wiley-Interscience, New York N.Y., 1993.

In another aspect, the present application provides one or more vectors, which comprises one or more nucleic acid molecules according to the present application. Each vector may include one or more of the nucleic acid molecules. In addition, the vector may further include another gene, for example, a marker gene that allows selection of the vector in an appropriate host cell and under an appropriate condition. In addition, the vector may further include an expression control element that allows correct expression of a coding region in an appropriate host. Such a control element is well known to those skilled in the art, and may include, for example, a promoter, a ribosome binding site, an enhancer, and other control elements that regulate gene transcription or mRNA translation. In certain embodiments, the expression control sequence is a regulatable element. The specific structure of the expression control sequence may vary depending on species or the function of cell types, but generally includes a 5' non-transcribed sequence and 5' and 3' non-translated sequences involved in the initiation of transcription and translation, respectively, for example, a TATA box, a caped sequence, and a CAAT sequence. For example, the 5' non-transcribed expression control sequence may include a promoter region, and the promoter region may include a promoter sequence for transcriptional control of a functionally linked nucleic acid. The expression control sequence may further include an enhancer sequence or an upstream activator sequence. In the present application, a suitable promoter may include, for example, a promoter for SP6, T3 and T7 polymerases, a human U6 RNA promoter, a CMV promoter and an artificial hybrid promoter thereof (e.g., CMV), wherein a certain moiety of the promoter may be fused with a certain moiety of the gene promoter of another cellular protein (e.g., human GAPDH, and glyceraldehyde-3-phosphate dehydrogenase). The promoter may or may not include an additional intron. One or more nucleic acid molecules described herein may be operably linked to the expression control element. The vector may be, for example, a plasmid, cosmid, virus, phage, or other vectors commonly used in, for example, genetic engineering. For example, the vector is an expression vector.

In another aspect, the present application provides a cell, which may include the one or more nucleic acid molecules according to the present application, and/or the one or more vectors according to the present application. In certain embodiments, each type of or each cell may comprise one or one type of the nucleic acid molecules or the vectors according to the present application. In certain embodiments, each type of or each cell may comprise multiple (e.g., 2 or more) or multiple types (e.g., 2 or more types) of the nucleic acid molecules or the vectors according to the present application. For example, the vector according to the present application may be introduced into the cell, e.g., a eukaryotic cell, such as a plant-derived cell, a fungus, or a yeast cell. The vector according to the present application may be introduced into the cell by methods known in the art, for example, electroporation, lipofectine transfection, and lipofectamin transfection.

### Preparation method

In another aspect, the present application provides a method for preparing the multispecific molecule. The method may comprise: culturing the host cell according to the present application under a condition that allows expression of the multispecific molecule. For example, uses of appropriate culture media, appropriate temperature, and culture time are known methods to those skilled in the art.

The humanized antibody can be selected from any type of immunoglobulins, including IgM, IgD, IgG, IgA and IgE. In the present application, the antibody is an IgG antibody, and the IgG1 subtype is used. Likewise, either type of light chain can be used in the compounds and methods herein. In particular, κ, λ chains or variants thereof are useful in the compounds and methods of the present application.

The sequence of the DNA molecule of the multispecific molecule or its fragment of the present application can be obtained by conventional techniques, such as PCR amplification or genomic library screening. In addition, the coding sequences of the light chain and heavy chain can be fused together to form a single-chain antibody.

Once the sequence of interest is obtained, it can be produced in large quantities by using recombination. This is usually done by cloning it into a vector, then transferring it into cells, and then isolating the sequence of interest from the proliferated cells by conventional methods.

In addition, artificial synthesis can also be used to synthesize sequences of interest, especially when the fragment length is short. Typically, fragments with long sequences can be obtained by synthesizing multiple small fragments and then ligating them. The nucleic acid molecule can then be introduced into various existing DNA molecules (or vectors) and cells known in the art.

The present application further relates to a vector comprising the above-mentioned appropriate nucleic acid molecule and an appropriate promoter or control sequence. These vectors can be used to transform appropriate host cells to enable them to express proteins. The host cell can be a prokaryotic cell, such as a bacterial cell; or a lower eukaryotic cell, such as a yeast cell; or a higher eukaryotic cell, such as a mammalian cell. For example, animal cells can include (but are not limited to): CHO-S, CHO-K1, HEK-293 cells.

The steps of transforming cells with recombinant DNA according to the present application can be performed using techniques well known in the art. The obtained transformants can be cultured using conventional methods, and the transformants express the polypeptide encoded by the nucleic acid molecule of the present application. Depending on the cells used, culture is performed using a conventional culture medium under appropriate conditions. Typically, the transformed cells are cultured under conditions suitable for expression of the multispecific molecule of the present invention. The multispecific antibody of the present application is then purified using conventional immunoglobulin purification steps, such as protein A-Sepharose, hydroxyapatite chromatography, gel electrophoresis, dialysis, ion exchange chromatography, hydrophobic chromatography, molecular sieve chromatography or affinity chromatography, and other conventional separation and purification methods well known to those skilled in the art.

The resulting monoclonal antibodies or multispecific molecules can be identified by conventional means. For example, the binding specificity of the monoclonal antibody or multispecific antibody can be determined by immunoprecipitation or by an in vitro binding assay such as fluorescence activated cell sorting (FACS), radioimmunoassay (RIA), or enzyme-linked immunosorbent assay (ELISA).

### Pharmaceutical composition

In another aspect, the present application further provides a composition. In some cases, the composition may be a pharmaceutical composition, which contains the multispecific antibody of the present application and a pharmaceutically acceptable carrier. Generally, these materials are formulated in a non-toxic, inert and pharmaceutically acceptable aqueous carrier medium. The formulated pharmaceutical composition can be administered via conventional routes.

The pharmaceutical composition according to the present application can be directly used to bind to IL-11 or TSLP protein molecules, and thus can be used to prevent and treat IL-11 or TSLP-related diseases. The pharmaceutical composition of the present application may contain a safe and effective amount of the antigen-binding protein according to the present application and a pharmaceutically acceptable adjuvant (which may include a carrier or excipient). The drug formulation should be compatible with the route of administration.

The multispecific antibodies or pharmaceutical compositions described herein can be formulated, dosed, and administered in a fashion consistent with the good medical practice. Considerations in this context include the particular disorder being treated, the particular mammal being treated, the clinical condition of the individual patient, the etiology of the disorder, the site of agent delivery, the method of administration, the schedule of administration and other factors known to medical practitioners.

### Use and method

In another aspect, the present application further provides a method for blocking the binding of TSLP to a receptor TSLPR/IL7Ra complex, comprising administering the multispecific molecule and/or the pharmaceutical combination.

In another aspect, the present application further provides a method for inhibiting TARC (thymus and activation-regulated chemokine) production, comprising administering the multispecific antibody and/or the pharmaceutical combination.

In another aspect, the present application further provides the use of the multispecific molecule, the pharmaceutical combination, the nucleic acid molecule, the vector, the cell and/or the pharmaceutical composition in preparing a medicament. The medicament is used for preventing, alleviating and/or treating a disease or disorder, such as an IL-11- related disease, such as a TSLP-related disease. In certain instances, the disease or disorder may be a fibrotic disease, an inflammatory disease, or a tumor.

In another aspect, the present application provides a method for preventing, alleviating and/or treating a disease or disorder, comprising administering to a patient in need thereof the multispecific molecule, the pharmaceutical combination, the nucleic acid molecule, the vector, the cell and/or the pharmaceutical composition according to the present application. In certain instances, the disease or disorder may be an IL-11- related disease, may be a TSLP- related disease, may be a fibrotic disease, an inflammatory disease or a tumor.

In another aspect, the present application provides the multispecific molecule, the pharmaceutical combination, the nucleic acid molecule, the vector, the cell and/or the pharmaceutical composition for use in the prevention, alleviation and/or treatment of diseases or disorders. In certain instances, the disease or disorder may be an IL-11- related disease, may be a TSLP- related disease, may be a fibrotic disease, an inflammatory disease or a tumor.

Without wishing to be bound by any theory, the following examples are only for illustrating the multispecific molecules, preparation methods and uses of the present application, and are not used to limit the scope of the invention of the present application.

### EXAMPLES

### Example 1 Sequence design of heavy chain and light chain of TSLP/IL-11 bispecific antibody

For the TSLP/IL-11 bispecific antibody in the present application, the monoclonal antibody targeting TSLP was derived from hybridoma technology. Mice were immunized by human TSLP protein, and the most desirable mice were selected to obtain spleen cells for fusion with SP2/0 myeloma cells. After monoclonal screening, functional activity and affinity testing, candidate molecules were obtained, and then humanized. In addition, the VHH nano single-chain antibody sequence targeting IL-11 was derived from alpacas immunized with human IL-11 protein and obtained through a phage display technology platform.

The structure of the TSLP/IL-11 multispecific molecule in the present application belongs to VHH-IgG, that is, the N-termini or C-termini of the two heavy chains of an IgG antibody targeting the TSLP antigen are both linked to a VHH fragment of another nanobody targeting the IL-11 antigen.

Among others, I700019, I700020, and I700021 antibodies are in a configuration in which VHH is linked to the N-terminus of the IgG antibody (as shown in Fig. 1A), and this type of bispecific antibody structure is abbreviated as IL11-VHH-Linker-TSLP-IgG; I700022, I700023, and I700024 antibodies are in a configuration in which VHH is linked to the C-terminus of the IgG antibody heavy chain (as shown in Fig. 1B), and this type of bispecific antibody structure is abbreviated as TSLP-IgG-Linker-IL-11-VHH.

The full length sequence of the heavy chain of I700019, SEQ ID NO: 58:

QVQLVESGGGLVQPGGSLRLSCAASGSIASIYTMAWYRQAPGKQRELVALSTNRGA
TDYADFVKGRFTISRDNAENTMYLQMNSLKPEDTAVYYCNGVASSWSTGFVASWGQ
GTQVTVSSGGGGSGGGGSQVQLVQSGAEVKKPGASVKVSCKASGYAFTNYLIEWVRQ
APGQGLEWMGIINPESGDTYYAQKFQGRVTMTADKSTSTAYMELSSLRSEDSAVYFCA
KESLTGSSYDYWGQGTTLTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTKVDKR
VEPKSCDKTHTCPPCPAPEAAGGPSVFLFPPKPKDTLMISRTPEVTCVVVDVSHEDPEVK

FNWYVDGVEVHNAKTKPREEQYNSTYRVVSVLTVLHQDWLNGKEYKCKVSNKALPAP

IEKTISKAKGQPREPQVYTLPPSREEMTKNQVSLTCLVKGFYPSDIAVEWESNGQPENNY

KTTPPVLDSDGSFFLYSKLTVDKSRWQQGNVFSCSVMHEALHNHYTQKSLSLSPG

The full length sequence of the heavy chain of I700020, SEQ ID NO: 59:

QVQLVESGGGLVQPGGSLRLSCAASGSIASIYTMAWYRQAPGKQRELVALSTNRGA
TDYADFVKGRFTISRDNAENTMYLQMNSLKPEDTAVYYCNGVASSWSTGFVASWGQ
GTQVTVSSGGGGSGGGGSQVQLQQSGAEVKKPGASVKVSCKASGYAFTNYLIEWVRQ
APGQGLEWMGVINPESGDTYYSQKFQGRVTMTADKSTSTAYMELSSLRSEDSAVYFCA
KESLTGSSYDYWGQGTTLTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTKVDKR
VEPKSCDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPG

The full length sequence of the heavy chain of I700021, SEQ ID NO: 60: 
QVQLVESGGGLVQPGGSLRLSCAASGSIASIYTMAWYRQAPGKQRELVALSTNRGA
TDYADFVKGRFTISRDNAENTMYLQMNSLKPEDTAVYYCNGVASSWSTGFVASWGQ
GTQVTVSSGGGGSGGGGSQVQLVQSGAEVKKPGASVKVSCKASGYAFTNYLIEWVRQ
APGQGLEWMGIINPESGDTYYAQKFQGRVTLTADKSTSTAYMELSSLRSEDSAVYYCA
KESLTGSSYDYWGQGTTLTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTKVDKR
VEPKSCDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPG
 Note: The underlined bold part is the CDR region

The full length sequence of the heavy chain of I700022, SEQ ID NO: 61:

QVQLVQSGAEVKKPGASVKVSCKASGYAFTNYLIEWVRQAPGQGLEWMGIINPES
GDTYYAQKFQGRVTMTADKSTSTAYMELSSLRSEDSAVYFCAKESLTGSSYDYWGQG
TTLTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKRVEPKSCDKTHTCPPCP
APEAAGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPGGGGGSGGGGSQVQLVESGG
GLVQPGGSLRLSCAASGSIASIYTMAWYRQAPGKQRELVALSTNRGATDYADFVKGRF
TISRDNAENTMYLQMNSLKPEDTAVYYCNGVASSWSTGFVASWGQGTQVTVSS

The full length sequence of the heavy chain of I700023, SEQ ID NO: 62:

QVQLQQSGAEVKKPGASVKVSCKASGYAFTNYLIEWVRQAPGQGLEWMGVINPES
GDTYYSQKFQGRVTMTADKSTSTAYMELSSLRSEDSAVYFCAKESLTGSSYDYWGQG
TTLTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKRVEPKSCDKTHTCPPCP
APEAAGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPGGGGGSGGGGSQVQLVESGG
GLVQPGGSLRLSCAASGSIASIYTMAWYRQAPGKQRELVALSTNRGATDYADFVKGRF
TISRDNAENTMYLQMNSLKPEDTAVYYCNGVASSWSTGFVASWGQGTQVTVSS

The full length sequence of the heavy chain of I700024, SEQ ID NO: 63:

QVQLVQSGAEVKKPGASVKVSCKASGYAFTNYLIEWVRQAPGQGLEWMGIINPES
GDTYYAQKFQGRVTLTADKSTSTAYMELSSLRSEDSAVYYCAKESLTGSSYDYWGQG
TTLTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKRVEPKSCDKTHTCPPCP
APEAAGGPSVFLFPPKPKDTLMISRTPEVTCVVVDVSHEDPEVKFNWYVDGVEVHNAKT

KPREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYS
KLTVDKSRWQQGNVFSCSVMHEALHNHYTQKSLSLSPGGGGGSGGGGSQVQLVESGG
GLVQPGGSLRLSCAASGSIASIYTMAWYRQAPGKQRELVALSTNRGATDYADFVKGRF
TISRDNAENTMYLQMNSLKPEDTAVYYCNGVASSWSTGFVASWGQGTQVTVSS

The full length sequence of the light chain of I700019, I700021, I700022, I700024, SEQ ID NO: 18:

DIQMTQSPSSLSASVGDRVTITCRASENIYSNLVWYQQKPGKAPQLLIYGARNLQSG
VPSRFSGSGSGTQFTLTISSLQPEDFATYYCQHFYETPLTFGTGTKLEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC

The full length sequence of the light chain of I700020, I700023, SEQ ID NO: 17: 
DIQMTQSPSSLSASVGDRVTITCRASENIYSNLVWYQQKPGKAPQLLVYGARNLQSG
VPSRFSGSGSGTQFSLTISSLQPEDFATYYCQHFYETPLTFGTGTKLEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC
 Note: The underlined bold part is the CDR region, which specifically is as follows:

| IL11-VHH CDR sequence (using Kabat numbering rules) | | | |
|---|---|---|---|
| Domain | | Sequence | SEQ ID NO. |
| | CDR1 | IYTMA | SEQ ID NO: 5 |
| VH | CDR2 | LSTNRGATDYADFVKG | SEQ ID NO: 6 |
| | CDR3 | VASSWSTGFVAS | SEQ ID NO: 7 |

| TSLP antibody CDR sequence (using IMGT numbering rules) | | | |
|---|---|---|---|
| Domain | | Sequence | SEQ ID NO. |
| VH | CDR1 | GYAFTNYL | SEQ ID NO: 19 |
| | CDR2 | INPESGDT | SEQ ID NO: 20 |
| | CDR3 | AKESLTGSSYDY | SEQ ID NO: 21 |
| VL | CDR1 | ENIYSN | SEQ ID NO: 22 |
| | CDR2 | GAR | SEQ ID NO: 23 |
| | CDR3 | QHFYETPLT | SEQ ID NO: 24 |

### Example 2 Expression and purification of TSLP/IL-11 bispecific antibody

The expression vector obtained in Example 1 was amplified by E. coli and a sufficient number of plasmids were prepared using an endotoxin-free plasmid extraction kit (Tiangen Biochemical Technology (Beijing) Co., Ltd., #DP117) for transient transfection and expression of bispecific antibodies. The host cells used for expression were CHO-S cells (Thermo Fisher, #R80007). By mixing the two prepared heavy chain carriers separately with light chain carriers and forming liposome complexes with polyetherimide (PEI, Polysciences, #24765-1), CHO-S cells were transfected and then cultured in an incubator for 5-7 days. The supernatant of the cell culture medium was collected by centrifugation and purified through a Protein A affinity chromatography column to obtain bispecific antibodies, and further the aggregation of proteins was determined by molecular exclusion chromatography.

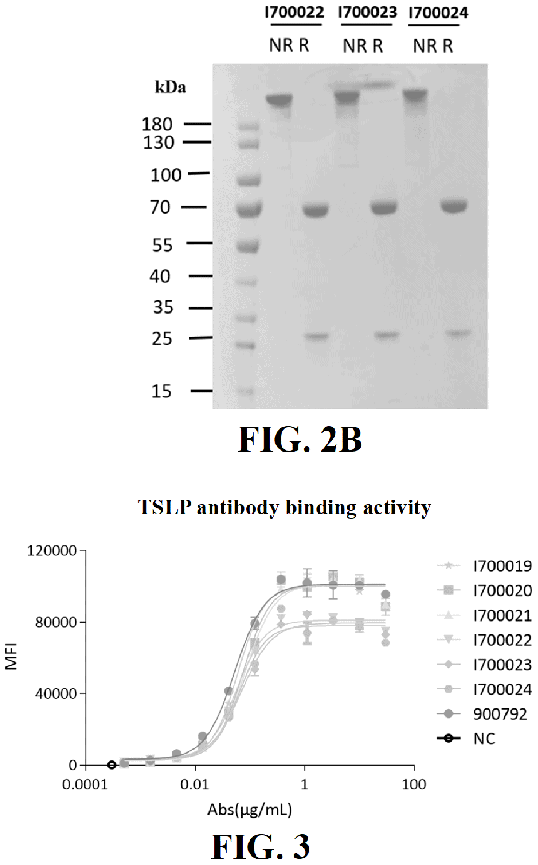

The expression, purification and detection of TSLP/IL-11 bispecific antibodies are shown in Figs. 2A, 2B and Table 1. The detection results show that the SEC purity of all the 6 TSLP/IL-11 bispecific antibodies reached 98% or more. The reduced and non-reduced SDS-PAGE images showed that the heavy chains and the light chains of all the 6 TSLP/IL-11 bispecific antibodies were not degraded, indicating that the purity and stability data of all the 6 TSLP/IL-11 bispecific antibodies were excellent.

**Table 1 SEC purity data of TSLP/IL-11 bispecific antibody**

| Antibody number | SEC purity (%) |
|---|---|
| I700019 | 99.34 |
| I700020 | 98.82 |
| I700021 | 99.47 |
| I700022 | 99.11 |
| I700023 | 98.63 |
| I700024 | 99.16 |

### Example 3 Detection of biological activity of humanized anti-human TSLP antibodies

### 3.1 Detection of the binding activity of anti-human TSLP antibody to TSLP-positive cells by using a flow cytometer

BaF3-huTSLP-3G11 cells were collected, resuspended in 1% BSA/PBS, diluted to 1×10⁶ cells/mL, and added to a 96-well U-shaped plate (Greiner, 780201) at 20 µL per well. The anti-TSLP antibody was diluted to 60 µg/mL with 1% BSA/PBS and then diluted 3-fold to 10 concentrations, for a total of 11 concentration gradients.

Anti-TSLP antibodies diluted in different concentrations (20 µL per well) were added to 96-well U-shaped plates and then incubated at room temperature for 30 min. At the end of incubation, 1% BSA/PBS was added to the 96-well U-shaped plate at 100 µL per well, and then the cells were collected by centrifugation at 300 g (Thermo Fisher SCIENTIFIC, Sorvall ST16/16R) for 3 min, the supernatant was removed, and the above washing steps were repeated 2 times. Then, 1:200 diluted R-Phycoerythrin AffiniPure Goat Anti-Human IgG, Fcγ Fragment antibody was added to the 96-well U-shaped plate, 20 µL per well, and incubated at room temperature for 15 min. At the end of incubation, 1% BSA/PBS was added to the 96-well U-shaped plate, 100 µL per well, and then the cells were collected by centrifugation at 300 g for 3 min, the supernatant was removed, and this step were repeated 3 times. 1% BSA/PBS was added to the 96-well U-shaped plate to resuspend the cells, 100 µL per well, and the fluorescence signal value was read using a flow cytometer.

The experimental results are shown in Fig. 3. The anti-TSLP antibody I700021 of the present disclosure substantially maintains the binding activity of the parental antibody 900792.

### 3.2 Detection of blocking activity of anti-human TSLP antibody on TSLP-TSLPR/IL7Ra mutual binding

Biotin-TSLP (Acro, TSP-H82EB-200) was diluted to 1.6 µg/mL with 1% BSA/PBS. The anti-TSLP antibody was diluted to 120 µg/mL with 1% BSA/PBS and then diluted 2-fold to 10 concentrations, for a total of 11 concentration gradients. Biotin-TSLP was mixed with anti-TSLP antibodies of different dilutions at a ratio of 1:1 and then added to a 96-well U-shaped plate (Greiner, 780201), 20 µL per well. Baf3-huTSLP/IL7Ra- 3H9-2 cells were collected, resuspended in 1% BSA/PBS, diluted to 1×10⁶ cells/mL, added to a 96-well U-shaped plate, 20 µL per well, and incubated at room temperature for 30 min. At the end of incubation, 1% BSA/PBS was added to the 96-well U-shaped plate, 100 µL per well, and then the cells were collected by centrifugation at 300 g for 3 min, the supernatant was removed, and the above washing step were repeated 1 time. Then, 1:200 diluted SA-APC antibody (BD, 554067) was added to the 96-well U-shaped plate, 20 µL per well, the system was mixed well, and incubated at room temperature for 30 min. At the end of incubation, 1% BSA/PBS was added to the 96-well U-shaped plate, 100 µL per well, and then the cells were collected by centrifugation at 300 g for 3 min, the supernatant was removed, and this step were repeated 1 time. 1% BSA/PBS was added to the 96-well U-shaped plate to resuspend the cells, 100 µL per well, and the fluorescence signal value was read using a flow cytometer.

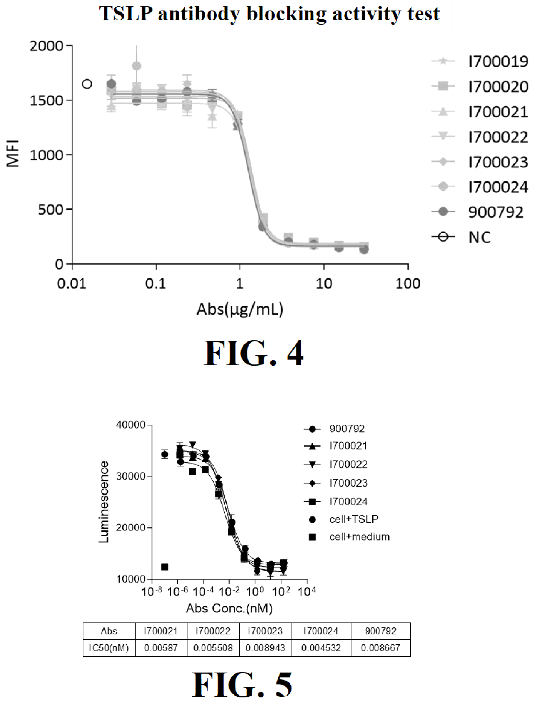

The experimental results are shown in Fig. 4. The anti-TSLP antibody I700021 of the present disclosure substantially maintains similar blocking activity as the parental antibody 900792.

### 3.3 Anti-human TSLP antibody blocks TSLP-induced activation of H_TSLP reporter cell line

H_TSLP reporter cell line (GM-C15572 from Jiman Biotechnology) cells were collected, resuspended in RPMI 1640 medium (Gibco Cat#A1049101) containing 10% FBS (Gibco, 10099141C), diluted to 3×10⁶ cells/mL, and added to a 96-well white plate (Corning, 3599) at 33 µL per well. The anti-TSLP antibody was diluted to 100 µg/mL with RPMI 1640 medium containing 10% FBS, and then diluted 10-fold to 8 concentrations, for a total of 9 concentration gradients. Antibodies with different concentration gradients were added into the 96-well white plate at 33 µL per well and incubated at room temperature for 1 h. Human TSLP (R127A, R130A) protein (Acro, Cat# TSP-H52Ha) was diluted to 4 ng/mL in RPMI 1640 medium containing 10% FBS, and added to the 96-well white plate at 33 µL per well. The 96-well plate was placed in a 37°C, 5% CO₂ incubator for culture for 23 h. After the incubation, the 96-well white plate was taken out and equilibrated at room temperature for 15 min. One-Glo reagent (Promega, E6120) was added at 100 µL per well, and full-wavelength fluorescence detection was performed using a microplate reader (MD SpectraMax^{®} i3x).

The experimental results are shown in Fig. 5. All the anti-human TSLP antibodies can inhibit the binding of human TSLP (R127A, R130A) protein to huTSLPR on the H_TSLP reporter cell line, and they are comparable to the parental TSLP antibodies in terms of the biological activity in inhibiting the binding of TSLP to TSLPR.

### 3.4 Anti-IL-11 antibody blocks IL-11-mediated activation of hIL11 effector reporter cells

hIL11 Effector Reporter Cell (Nanjing Cobioer Biosciences, CBP74114) reporter gene cell line is a Luciferase reporter gene cell line constructed based on the JAK1-STAT3 intracellular signaling pathway. IL-11 binds to the IL-11R receptor and recruits gp130 to form a ternary complex, which activates JAK, phosphorylates STAT3, and then transports it to the cell nucleus to activate the expression of luciferase. The reading value of luciferase-catalyzed luminescence of luciferin substrate represents the activation effect of the signal pathway and can therefore be used to evaluate the in vitro effects of IL-11-related drugs.

The hIL11 effector reporter cells were collected and resuspended in DMEM medium (Gibco, 11965092) containing 10% FBS (Gibco, 10099141C), diluted to 1.2×106 cells/mL, and added to a 96-well white plate (Corning, 3599) at 50 µL per well. The 96-well white plate was placed in a 37°C, 5% CO2 incubator for overnight culture. The anti-IL-11 antibody was diluted to 120 µg/mL with DMEM medium containing 10% FBS, and then diluted 3-fold to 10 concentrations, for a total of 11 concentration gradients. Antibodies with different concentration gradients were added into the 96-well white plate at 25 µL per well. Human IL-11 (novoprotein, C006) was diluted to 1 ng/mL using DMEM0 medium containing 10% FBS, and added to the 96-well white plate at 25 µL per well. The 96-well white plate was placed in a 37°C, 5% CO₂ incubator for culture for 6 h. After the incubation, the 96-well white plate was taken out and equilibrated at room temperature for 15 min. Bright-Glo reagent (Promega, E2620) was added at 100 µL per well, and full-wavelength fluorescence detection was performed using a microplate reader (MD SpectraMax^{®} i3x).

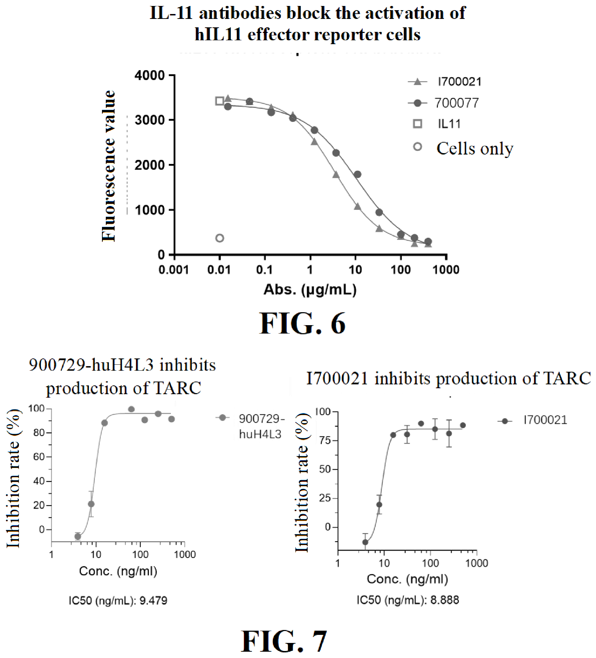

The experimental results are shown in Fig. 6. The IC50 of 700077 (anti-IL-11 monoclonal antibody) in blocking IL-11-induced hIL11 effector reporter cell activation is 10.41 µg/mL. Correspondingly, the IC50 of I700021 in blocking reporter gene cell line activation is 3.457 µg/mL. Therefore, the anti-IL-11 and TSLP bispecific antibody I700021 of the present disclosure basically maintains similar blocking activity as the parental IL-11 monoclonal antibody 700077.

### 3.5 Anti-human TSLP antibody blocks TSLP-stimulated production of TARC by PBMC

TSLP can stimulate dendritic cells or monocytes to produce the chemokine TARC (thymus and activation-regulated chemokine), thereby recruiting type 2 helper T cells and causing type 2 inflammatory response, while TARC is not produced in the resting state. In an in vitro culture system, dendritic cells and monocytes in PBMC are stimulated by TSLP, and the content of TARC produced is detected. After adding anti-TSLP antibodies to neutralize the added TSLP, the production of TARC is blocked, which represents a dose-dependent relationship with the anti-TSLP antibodies with concentration gradient, thereby evaluating the neutralizing activity of the antibody.

Experimental method: Anti-TSLP antibody was diluted to 2 µg/mL, then diluted 2-fold to a total of 7 concentrations, and added to a 96-well plate at 50 µL/well, and the final concentration was 0.5 µg/mL. TSLP was diluted to 10 ng/mL and added to the above-mentioned well plate, 50 µL/well, mixed and incubated for 30 min, with a final concentration of 2.5 ng/mL. The cells were resuscitated, centrifuged at 500 g for 5 min, and the supernatant was removed. The cells were resuspended in the complete medium and counted, adjusted to density to 2x10⁶ cells/mL, and add 100 µL/well to the cell plate. The incubated antigen-antibody mixture was added to the cells, 100 µL/well, and cultured in a 37°C 5% CO₂ incubator for 48 h, and then centrifuged to obtain the supernatant. The TARC content in the cell culture supernatant was detected by ELISA.

The experimental results are shown in Fig. 7. I700021 can effectively inhibit the content of CCL17 produced by PBMC stimulated by TSLP, and the IC50 of the effect is equivalent to that of the parental TSLP monoclonal antibody.

### 3.6 I700021 antibody affinity test

### 3.6.1 Affinity test of I700021 antibody for human, monkey, rat and mouse TSLPs

Test materials and instruments:

| Name | Manufacture | Cat# |
|---|---|---|
| Human TSLP, His Tag | ACRO Biosystems | TSP-H52Ha |
| Cynomolgus TSLP (R127A, R130S), His Tag | ACRO Biosystems | TSLP-C52H4 |
| Rat TSLP, His Tag | ACRO Biosystems | TSP-R52H3 |
| Mouse TSLP, His Tag | ACRO Biosystems | TSP-M52H8 |
| Amine Coupling Kit | Cytiva | BR-1000-50 |
| HBS-EP+ Buffer (10X) | Cytiva | BR-1006-69 |
| His Capture Kit, Type 2 | Cytiva | 29-2346-02 |
| Series S Sensor Chip CM5 | Cytiva | BR-1005-30 |
| 10mM Glycine, pH1.5 | Cytiva | BR-1003-54 |
| BIACORE | Cytiva | Biacore 8K |

Experimental method (SPR):
The sample chamber and flow cell temperature of Biacore 8k was set to be 25°C, and the data collection frequency was 10 Hz. Anti-His Antibody is immobilized on the Series S Sensor Chip CM5 chip by amino coupling using the Amine Coupling Kit to obtain the Anti-His Series CM5 chip. Anti-His Series CM5 chip was used to respectively capture TSLPs from human, monkey, rat and mouse as ligands. The antibody sample was diluted with HBS-EP+, pH 7.4 buffer to reach a series of gradient concentrations of 20 nM, 10 nM, 5 nM, 2.5 nM, 1.25 nM, 0.625 nM, 0.3125 nM, and 0.78 nM as the analyte, and HBS-EP+, pH 7.4 buffer was used as the 0 concentration for background deduction. Multi-cycle kinetics was used with the analysis flow rate set at 30 µL/min, binding time 120 s, dissociation time 600 s, and regeneration at 10 mM glycine (pH 1.5) at 50 µL/min for 60 s. 1:1 binding mode and Fit Local Kinetics mode was used for analyzing data.

**Table 2 Results of affinity test of I700021 antibody for human, monkey, rat and mouse TSLPs**

| **Sample** | **Antigen** | **ka (1/Ms)** | **kd (1/s)** | **KD (M)** |
|---|---|---|---|---|
| | Human TSLP | 5.65×10⁵ | 9.87×10⁻⁵ | 1.75×10⁻¹⁰ |
| I700021 | Cynomolgus TSLP | 3.08×10⁶ | 5.80×10⁻³ | 1.88×10⁻⁰⁹ |
| | Rat TSLP | NB | NB | NB |
| | Mouse TSLP | NB | NB | NB |

| | | | | |
|---|---|---|---|---|
| Note: NB refers to No Binding | | | | |

The results of affinity constants (KD(M)) for various species showed that the affinity of the humanized anti-TSLP/IL-11 bispecific monoclonal antibody I700021of the present invention for human TSLP is on the order of 10⁻¹¹, and it has extremely strong affinity for human antigens. Its affinity for monkey TSLP is on the order of 10⁻⁰⁹. Furthermore, it does not bind to rat and mouse TSLPs.

### 3.6.2 Affinity test of I700021antibody for human and mouse IL-11

Test materials and instruments:

| Name | Manufacture | Cat# |
|---|---|---|
| Human IL-11, No Tag | Sino Biological | 12225-HNCE |
| Mouse IL-11, No Tag | Sino Biological | 50117-MNCE |
| HBS-EP+ Buffer (10X) | Cytiva | BR-1006-69 |
| Series S Sensor Chip Protein A | Cytiva | 29-1275-56 |
| 10mM Glycine, pH1.5 | Cytiva | BR-1003-54 |
| BIACORE | Cytiva | T200 |

Experimental method (SPR):
The temperature of the sample chamber and flow cell of Biacore T200 was set to be 25°C, and the data collection frequency was 10 Hz. The Series S Protein A chip captured the antibody samples to 300RU as ligands, respectively. Human and mouse IL-11 antigens were diluted by HBS-EP+ buffer, pH 7.4 to reach a series of gradient concentrations of 50 nM, 25 nM, 12.5 nM, 6.25 nM, 3.125 nM, 1.56 nM, 0.78 nM, and 0.39 nM as analytes, and HBS-EP+ buffer, pH 7.4 was used as 0 concentrations for background deduction. Multi-cycle kinetics was used with the analysis flow rate set at 30 µL/min, binding time 120 s, dissociation time 600 s, and regeneration at 10 mM glycine (pH 1.5) at 50 µL/min for 60 s. 1:1 binding mode and Fit Local Kinetics mode was used for analyzing data.

**Table 3 Results of affinity test of I700021antibody for human and mouse IL-11**

| **Sample** | **Antigen** | **ka (1/Ms)** | **kd (1/s)** | **KD (M)** |
|---|---|---|---|---|
| I700021 | Human IL-11 | 2.02×10⁵ | 9.26×10⁻⁴ | 4.58×10⁻⁰⁹ |
| | Mouse IL-11 | 4.51×10⁵ | 8.64×10⁻⁴ | 1.91×10⁻⁰⁹ |

| | | | | |
|---|---|---|---|---|
| Note: NB refers to No Binding | | | | |

The results of affinity constants (KD(M)) for various species showed that the affinity of the humanized anti-TSLP/IL-11 bispecific monoclonal antibody I700021of the present invention for either human or mouse IL-11 is on the order of 10⁻⁰⁹, and it has extremely strong affinity for human and mouse antigens.

### 3.6.3 Affinity test of I700021 antibody and rat IL-11

Test materials and instruments:

| Name | Manufacture | Cat# |
|---|---|---|
| Rat TSLP, His Tag | ACRO Biosystems | TSP-R52H3 |
| PBS | BasalMedia | B320KJ |
| Polysorbate 20 | Sigma-Aldrich | 44112 |
| SA Sensor | Sartorius | 18-5021 |
| Chromalink biotin354 (sulfo NHS) | Vector Laboratories | B-9007-105K |
| Octet | Sartorius | RH96 |

Experimental method (BLI):
An SA sensor was loaded with 5 µg/mL biotinylated sample as ligand and a Rat IL-11 solution diluted by gradient as analyte for kinetic detection. The kinetic constant was analyzed under the following conditions: temperature 30°C, shaking rate: 1000rpm, association: 360 s, dissociation: 360 s, 1:1 Binding Model Global Fit.

**Table 4 Results of affinity test of I700021 antibody and rat IL-11**

| **Sample** | **Antigen** | **R²** | **ka (1/Ms)** | **kd (1/s)** | **KD (M)** |
|---|---|---|---|---|---|
| I700021 | Rat IL-11 | 0.9999 | 8.450×10⁴ | 4.773×10⁻⁵ | 5.649×10⁻¹⁰ |

| | | | | | |
|---|---|---|---|---|---|
| Note: NB refers to No Binding | | | | | |

The results of affinity constants (KD(M)) for various species showed that the affinity of the humanized anti-TSLP/IL-11 bispecific monoclonal antibody I700021 of the present invention for rat IL-11 is on the order of 10⁻¹⁰, and it has extremely strong affinity for rat antigens.

### 3.6.4 Affinity test of I700021 antibody and monkey IL-11

Test materials and instruments:

| Name | Manufacture | Cat# |
|---|---|---|
| Cynomolgus IL-11 Protein | Sino biological | 90925-CNCE |
| Amine Coupling Kit | Cytiva | BR-1000-50 |
| HBS-EP+ Buffer (10X) | Cytiva | BR-1006-69 |
| Series S Sensor Chip CM5 | Cytiva | BR-1005-30 |
| 10mM Glycine, pH1.5 | Cytiva | BR-1003-54 |
| BIACORE | Cytiva | Biacore 8K |

Experimental method (SPR):
The sample chamber and flow cell temperature of Biacore 8k was set to be 25°C, and the data collection frequency was 10 Hz. Cynomolgus IL-11 was immobilized as a ligand on a Series S Sensor Chip CM5 chip by amino coupling using Amine Coupling Kit. Monkey IL-11 was diluted by HBS-EP+ buffer, pH 7.4 to reach a series of gradient concentrations of 20 nM, 10 nM, 5 nM, 2.5 nM and 1.25 nM as analytes, and HBS-EP+ buffer, pH 7.4 was used as 0 concentration for background deduction. Single-cycle kinetics was used with the analysis flow rate set at 30 µL/min, binding time 120 s, dissociation time 600 s, and regeneration at 10 mM glycine (pH 1.5) at 50 µL/min for 60 s. 1:1 binding mode and Fit Local Kinetics mode was used for analyzing data.

**Table 5 Results of affinity test of I700021 antibody and monkey IL-11**

| **Sample** | **Antigen** | **ka (1/Ms)** | **kd (1/s)** | **KD (M)** |
|---|---|---|---|---|
| I700021 | Cynomolgus IL-11 | 1.22×10⁵ | 3.54×10⁻⁴ | 2.90×10⁻⁰⁹ |

| | | | | |
|---|---|---|---|---|
| Note: NB refers to No Binding | | | | |

The results of affinity constants (KD(M)) showed that the affinity of the humanized anti-TSLP/IL-11 bispecific monoclonal antibody I700021 of the present invention for monkey IL-11 is on the order of 10⁻⁰⁹, and it has extremely strong affinity for monkey antigens.

### 3.7 Detection of the inhibitory effect of I700021 antibody on the fibrotic activity of MRC-5 cells

Transforming growth factor-β (TGF-β) can transform the phenotype of normal fibroblasts to differentiate and produce substances such as extracellular matrix and promote further fibrosis of tissues. Interleukin 11 (IL-11) has also been widely reported to be produced downstream of the TGF-β pathway and plays an important role in promoting fibrosis. It can produce inflammatory effects and is associated with a variety of allergic diseases, chronic inflammation, autoimmune diseases and cancer. After TGF-β induction, MRC-5 (human embryonic lung fibroblasts) can differentiate into myofibroblasts and upregulate the expression of fibrosis-related genes such as fibronectin (FN) and α-SMA (its encoding gene is ACTA2). In order to explore the potential effect of I700021 antibody in inhibiting fibrosis-related diseases, this study used MRC-5 as a carrier and detected the ability of the antibody to block the gene expression of the extracellular matrix component FN and the myofibroblast marker gene ACTA2 after TGF-β induction.

Experimental method: MRC-5 cells in the logarithmic growth phase in the culture flask were resuspended and cultured in a six-well plate at a cell number of 5×10⁵ per well for 8 h. After the cells became adherent, the culture medium (without FBS) was replaced and the cells were starved for 16 h. The final concentration of TGF-β was 10 ng/mL and the final concentration of the antibody was 100 nM. The relevant reagents were prepared in complete culture medium containing FBS. The blank group (Ctrl group) was complete culture medium, and each test condition was in triplicate. The original starvation medium of the starved cells was discarded, the above-prepared solution was added to the cells. The cells were cultured in an incubator for 24 h, collected into EP tubes without RNase, and centrifuged to obtain cell pellets. After total RNA was extracted according to the instructions of the RNeasy Micro kit, the extracted RNA was purified using gDNA eraser. Reverse transcription was performed according to the instructions of the PrimeScript^{™} RT reagent Kit to obtain cDNA. The cDNA was amplified by PCR using Fast Start Universal SYBR Green Master (Rox) as a fluorescent marker, human GAPDH as a housekeeping gene and primers for FN and ACTA2. Gene expression was represented as fold change and calculated using the 2^-ΔΔCt formula, where each value was normalized to the mean in the control group.

The expression levels of fibrosis-related genes FN and ACTA2 in MRC-5 cells were used as evaluation criteria to detect the inhibitory abilities of antibody I700021, parental anti-TSLP monoclonal antibody and anti-IL-11 monomer on TGF-β-induced fibrosis.

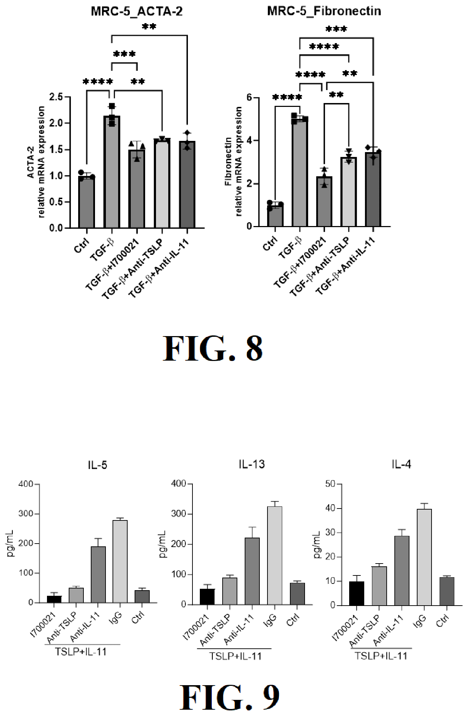

The experimental results are shown in Fig. 8. TGF-β induction can significantly increase the expression levels of the two genes, and the 3 antibodies tested can significantly inhibit the expression of the 2 genes. Moreover, the inhibitory effect of the bispecific antibody I700021 on the gene expression levels of ACTA2 and fibronectin was significantly stronger than that of the two parental monoclonal antibodies.

### 3.8 Detection of the pro-inflammatory effects of TH2 induced by TSLP and IL-11 by using I700021 antibody

The aim of this study was to evaluate the biological activity of I700021 antibody on TSLP- and IL-11-induced TH2 cytokine release in a PBMC system. The study found that IL-11 can promote PBMC to produce IL-33, while the combined stimulation of TSLP and IL-33 can more effectively induce PBMC to produce TH2 type cytokines, including IL-5 and IL-13. The I700021 antibody inhibits the production of TH2 cytokines by blocking the interaction of IL-11 and TSLP with PBMCs. Its ability to block TSLP- and IL-11-induced TH2 cytokine release was evaluated.

Experimental method: The cell suspension density of PBMC cells was adjusted to 2×10⁶ cells/mL and plated into a 96-well U-bottom plate at 50 µL/well, i.e. 1×10⁵ cells per well. The human TSLP stock solution was diluted into a 40 ng/mL working solution with the assay medium, and added to the sample wells of the 96-well plate after incubation at 50 µL/well (final concentration 10 ng/mL). The human IL-11 stock solution was diluted into a 400 ng/mL working solution with the assay medium, and added to the sample wells of the 96-well plate after incubation at 50 µL/well (final concentration 100 ng/mL). The antibody to be tested was diluted to 600 nM in the assay medium (final concentration 150 nM). The 96-well U-bottom plate was then placed in a 37°C, 5% CO₂ incubator and incubated for 3 days. The anti-CD3 antibody stock solution was diluted into a 4 µg/mL working solution with the assay medium and added to the washed cells. The cells were placed in a 37°C, 5% CO₂ incubator and further incubated for 1 day. 25 µL of the supernatant was taken for cytokine detection. TH2 cytokines were detected by flow cytometry using the LEGENDplex kit. GraphPad Prism 8 software was used to plot the sample working concentration - converted TH2 cytokine concentration.

The experimental results are shown in Fig. 9, showing that the combined stimulation of TSLP and IL-11 is conductive to inducing PBMC to produce a large amount of TH2-type cytokines (including IL-5, IL-13 and IL-4). Moreover, compared with the two parental monoclonal antibodies, the bispecific antibody can produce more TH2-type cytokines (including IL-5, IL-13 and IL-4) and has better inhibitory effects.

## Claims

1. A multispecific molecule comprising a first binding domain capable of targeting IL-11 and a second binding domain capable of targeting TSLP, the multispecific molecule having one or more of the following properties:
(1) being capable of binding a TSLP protein with a KD value of 1×10⁻⁹M or less, wherein the KD value is determined by surface plasmon resonance;
(2) being capable of binding an IL-11 protein with a KD value of 1×10⁻⁹M or less, wherein the KD value is determined by surface plasmon resonance;
(3) being capable of blocking the binding of TSLP to a receptor TSLPR/IL7Ra complex; and
(4) being capable of inhibiting TARC (Thymus and Activation-Regulated Chemokine) production.

2. The multispecific molecule according to claim 1, wherein the first binding domain capable of targeting IL-11 comprises an antibody or an antigen-binding fragment thereof.

3. The multispecific molecule according to any one of claims 1-2, wherein the antigen-binding fragment is selected from the group consisting of Fab, Fab', F(ab)₂, Fv fragment, F(ab'), scFv,
di-scFv, VHH and dAb fragment.

4. The multispecific molecule according to any one of claims 1-2, wherein the antibody is selected from the group consisting of a monoclonal antibody, a single chain antibody, a chimeric antibody, a multispecific antibody, a humanized antibody, a fully human antibody, a nanobody, and/or a heavy chain antibody.

5. The multispecific molecule according to any one of claims 1-4, wherein the first binding domain capable of targeting IL-11 comprises HCDR3, HCDR2 and HCDR1 of a heavy chain variable region VH; wherein the amino acid sequence of the HCDR3 is as set forth in SEQ ID NO: 7, the amino acid sequence of the HCDR2 is as set forth in SEQ ID NO: 6, and the amino acid sequence of the HCDR1 is as set forth in SEQ ID NO: 5.

6. The multispecific molecule according to any one of claims 1-5, wherein the first binding domain capable of targeting IL-11 comprises an antibody heavy chain variable region VH, and the VH comprises an amino acid sequence as set forth in SEQ ID NO: 4.

7. The multispecific molecule according to any one of claims 1-6, wherein the first binding domain capable of targeting IL-11 is VHH.

8. The multispecific molecule according to claim 7, wherein the VHH comprises an amino acid sequence as set forth in SEQ ID NO: 4.

9. The multispecific molecule according to any one of claims 1-8, wherein the first binding domain capable of targeting IL-11 comprises an amino acid sequence as set forth in SEQ ID NO: 4.

10. The multispecific molecule according to any one of claims 1-4, wherein the first binding domain capable of targeting IL-11 is selected from sequences known in the prior art.

11. The multispecific molecule according to any one of claims 1-4 and 10, wherein the first binding domain capable of targeting IL-11 comprises an antibody heavy chain variable region VH and/or an antibody light chain variable region VL.

12. The multispecific molecule according to any one of claims 1-4 and 10-11, wherein the first binding domain capable of targeting IL-11 comprises HCDR3, HCDR2 and HCDR1 of a heavy chain variable region VH; wherein the HCDR1 can be selected from sequences as set forth in SEQ ID NOs: 64, 65, 95, 104, 110 and 116, the HCDR2 can be selected from sequences as set forth in SEQ ID NOs: 66, 72, 73, 74, 96, 105, 111 and 117, and the HCDR3 can be selected from sequences as set forth in SEQ ID NOs: 67, 68, 97, 106, 112 and 118.

13. The multispecific molecule according to any one of claims 1-4 and 10-12, wherein the first binding domain capable of targeting IL-11 comprises LCDR3, LCDR2 and LCDR1 of a light chain variable region VL; wherein the LCDR1 can be selected from sequences as set forth in SEQ ID NOs: 69, 70, 71, 98, 101, 107, 113 and 119, the LCDR2 can be selected from sequences as set forth in SEQ ID NOs: 72, 73, 99, 102, 108, 114 and 120, and the LCDR3 can be selected from sequences as set forth in SEQ ID NOs: 74-94, 100, 103, 109, 115 and 121.

14. The multispecific molecule according to any one of claims 1-4 and 10-13, wherein the first binding domain capable of targeting IL-11 comprises an antibody heavy chain variable region VH, and the VH may comprise an amino acid sequence as set forth in SEQ ID NOs: 122-134.

15. The multispecific molecule according to any one of claims 1-4 and 10-14, wherein the first binding domain capable of targeting IL-11 comprises an antibody light chain variable region VL, and the VL may comprise an amino acid sequence as set forth in SEQ ID NOs: 135-177.

16. The multispecific molecule according to any one of claims 1-4 and 10-15, wherein the first binding domain capable of targeting IL-11 comprises an antibody heavy chain constant region.

17. The multispecific molecule according to any one of claims 1-4 and 10-16, wherein the first binding domain capable of targeting IL-11 comprises an antibody light chain constant region.

18. The multispecific molecule according to any one of claims 1-17, wherein the second binding domain capable of targeting TSLP comprises an antibody or an antigen-binding fragment thereof.

19. The multispecific molecule according to any one of claims 2-18, wherein the antigen-binding fragment is selected from the group consisting of Fab, Fab', F(ab)₂, Fv fragment, F(ab'), scFv, di-scFv, VHH and dAb.

20. The multispecific molecule according to any one of claims 2-19, wherein the antibody is selected from the group consisting of a monoclonal antibody, a single chain antibody, a chimeric antibody, a multispecific antibody, a humanized antibody, and a fully human antibody.

21. The multispecific molecule according to any one of claims 1-20, wherein the second binding domain capable of targeting TSLP comprises HCDR1, HCDR2 and HCDR3 of a heavy chain variable region VH; wherein the amino acid sequence of the HCDR1 is as set forth in SEQ ID NO: 19, the amino acid sequence of the HCDR2 is as set forth in SEQ ID NO: 20, and the amino acid sequence of the HCDR3 is as set forth in SEQ ID NO: 21.

22. The multispecific molecule according to any one of claims 1-21, wherein the second binding domain capable of targeting TSLP comprises an antibody heavy chain variable region VH, wherein the amino acid sequence of the VH is as set forth in SEQ ID NO: 49, SEQ ID NO: 50, SEQ ID NO: 51, SEQ ID NO: 52 or SEQ ID NO: 53.

23. The multispecific molecule according to any one of claims 1-22, wherein the second binding domain capable of targeting TSLP comprises LCDR1, LCDR2 and LCDR3 of a light chain variable region VL; wherein the amino acid sequence of the LCDR1 is as set forth in SEQ ID NO: 22, the amino acid sequence of the LCDR2 is as set forth in SEQ ID NO: 23 (GAR), and the amino acid sequence of the LCDR3 is as set forth in SEQ ID NO: 24.

24. The multispecific molecule according to any one of claims 1-23, wherein the second binding domain capable of targeting TSLP comprises an antibody light chain variable region VL, wherein the amino acid sequence of the VL is as set forth in SEQ ID NO: 54, SEQ ID NO: 55, SEQ ID NO: 56 or SEQ ID NO: 57.

25. The multispecific molecule according to any one of claims 1-24, wherein the second binding domain capable of targeting TSLP comprises an amino acid sequence selected from any one of the following groups of VH and VL:
1) VH: SEQ ID NO: 53, VL: SEQ ID NO: 57;
2) VH: SEQ ID NO: 49, VL: SEQ ID NO: 54;
3) VH: SEQ ID NO: 49, VL: SEQ ID NO: 55;
4) VH: SEQ ID NO: 49, VL: SEQ ID NO: 56;
5) VH: SEQ ID NO: 50, VL: SEQ ID NO: 54;
6) VH: SEQ ID NO: 50, VL: SEQ ID NO: 55;
7) VH: SEQ ID NO: 50, VL: SEQ ID NO: 56;
8) VH: SEQ ID NO: 51, VL: SEQ ID NO: 54;
9) VH: SEQ ID NO: 51, VL: SEQ ID NO: 55;
10) VH: SEQ ID NO: 51, VL: SEQ ID NO: 56;
11) VH: SEQ ID NO: 52, VL: SEQ ID NO: 54;
12) VH: SEQ ID NO: 52, VL: SEQ ID NO: 55; and
13) VH: SEQ ID NO: 52, VL: SEQ ID NO: 56.

26. The multispecific molecule according to any one of claims 1-25, wherein the second binding domain capable of targeting TSLP comprises an antibody heavy chain constant region.

27. The multispecific molecule according to claim 26, wherein the antibody heavy chain constant region is derived from a human IgG heavy chain constant region.

28. The multispecific molecule according to any one of claims 26-27, wherein the antibody heavy chain constant region is derived from a human IgG1 heavy chain constant region.

29. The multispecific molecule according to any one of claims 1-28, wherein the second binding domain capable of targeting TSLP comprises an antibody light chain constant region.

30. The multispecific molecule according to claim 29, wherein the antibody light chain constant region is derived from a human Igκ constant region.

31. The multispecific molecule according to any one of claims 1-30, wherein the second binding domain capable of targeting TSLP comprises an antibody heavy chain, said antibody heavy chain comprises an amino acid sequence as set forth in SEQ ID NO: 12, SEQ ID NO: 13, SEQ ID NO: 14 or SEQ ID NO: 15.

32. The multispecific molecule according to any one of claims 1-31, wherein the second binding domain capable of targeting TSLP comprises an antibody light chain, said antibody light chain comprises an amino acid sequence as set forth in SEQ ID NO: 16, SEQ ID NO: 17 or SEQ ID NO: 18.

33. The multispecific molecule according to any one of claims 1-32, wherein the second binding domain capable of targeting TSLP can be a sequence known in the prior art.

34. The multispecific molecule according to any one of claims 1-33, wherein the second binding domain capable of targeting TSLP can be Tezepelumab.

35. The multispecific molecule according to any one of claims 1-33, wherein the second binding domain capable of targeting TSLP comprises HCDR1, HCDR2 and HCDR3 of a heavy chain variable region VH; wherein the amino acid sequence of the HCDR1 is as set forth in SEQ ID NO: 178, the amino acid sequence of the HCDR2 is as set forth in SEQ ID NO: 179, and the amino acid sequence of the HCDR3 is as set forth in SEQ ID NO: 180.

36. The multispecific molecule according to any one of claims 1-33 and 35, wherein the second binding domain capable of targeting TSLP comprises LCDR3, LCDR2 and LCDR1 of a light chain variable region VL; wherein the amino acid sequence of the LCDR1 is as set forth in SEQ ID NO: 181, the amino acid sequence of the LCDR2 is as set forth in SEQ ID NO: 182, and the amino acid sequence of the LCDR3 is as set forth in SEQ ID NO: 183.

37. The multispecific molecule according to any one of claims 1-33 and 35-36, wherein the second binding domain capable of targeting TSLP comprises an antibody heavy chain variable region VH, and the VH comprises an amino acid sequence as set forth in SEQ ID NO: 184.

38. The multispecific molecule according to any one of claims 1-33 and 35-37, wherein the second binding domain capable of targeting TSLP comprises an antibody light chain variable region VL, and the VL comprises an amino acid sequence as set forth in SEQ ID NO: 185.

39. The multispecific molecule according to any one of claims 1-33 and 35-38, wherein the second binding domain capable of targeting TSLP comprises an antibody heavy chain constant region.

40. The multispecific molecule according to any one of claims 1-33 and 35-39, wherein the second binding domain capable of targeting TSLP comprises an antibody light chain constant region.

41. The multispecific molecule according to any one of claims 1-33 and 35-40, wherein the second binding domain capable of targeting TSLP comprises HCDR1, HCDR2 and HCDR3 of a heavy chain variable region; wherein the amino acid sequence of the HCDR1 is as set forth in SEQ ID NO: 186, the amino acid sequence of the HCDR2 is as set forth in SEQ ID NO: 187, and the amino acid sequence of the HCDR3 is as set forth in SEQ ID NO: 188.

42. The multispecific molecule according to any one of claims 1-33 and 35-41, wherein the second binding domain capable of targeting TSLP comprises HCDR1, HCDR2 and HCDR3 of a heavy chain variable region; wherein the amino acid sequence of the HCDR1 is as set forth in SEQ ID NO: 189, the amino acid sequence of the HCDR2 is as set forth in SEQ ID NO: 190, and the amino acid sequence of the HCDR3 is as set forth in SEQ ID NO: 191.

43. The multispecific molecule according to any one of claims 1-33 and 35-42, wherein the second binding domain capable of targeting TSLP comprises an antibody heavy chain variable region VH, and the VH comprises an amino acid sequence as set forth in SEQ ID NO: 192.

44. The multispecific molecule according to any one of claims 1-33 and 35-43, wherein the second binding domain capable of targeting TSLP comprises an antibody heavy chain variable region VH, and the VH comprises an amino acid sequence as set forth in SEQ ID NO: 193.

45. The multispecific molecule according to any one of claims 1-44, wherein the first binding domain capable of targeting IL-11 and the second binding domain capable of targeting TSLP are directly or indirectly linked.

46. The multispecific molecule according to any one of claims 1-45, wherein a C-terminus of the first binding domain capable of targeting IL-11 is linked to an N-terminus of the second binding domain capable of targeting TSLP.

47. The multispecific molecule according to claim 46, wherein a C-terminus of the first binding domain capable of targeting IL-11 is linked to an N-terminus of the heavy chain variable region VH of the second binding domain capable of targeting TSLP.

48. The multispecific molecule according to any one of claims 1-47, wherein an N-terminus of the first binding domain capable of targeting IL-11 is linked to a C-terminus of the second binding domain capable of targeting TSLP.

49. The multispecific molecule according to claim 48, wherein an N-terminus of the first binding domain capable of targeting IL-11 is linked to a C-terminus of the heavy chain constant region of the second binding domain capable of targeting TSLP.

50. The multispecific molecule according to any one of claims 1-49, wherein the first binding domain capable of targeting IL-11 is linked to the second binding domain capable of targeting TSLP through a linker.

51. The multispecific molecule according to claim 50, wherein the linker comprises a peptide linker.

52. The multispecific molecule according to any one of claims 50-51, wherein the linker comprises an amino acid sequence of (GGGGS)n, wherein n is any positive integer from 0-10.

53. The multispecific molecule according to any one of claims 50-52, wherein the linker comprises an amino acid sequence as set forth in GGGGSGGGGS (SEQ ID NO: 3).

54. The multispecific molecule according to any one of claims 50-53, wherein the multispecific molecule comprises two first binding domains capable of targeting IL-11.

55. The multispecific molecule according to claim 54, wherein the two first binding domains capable of targeting IL-11 are linked directly or indirectly to the N-termini of two heavy chains of the second binding domain capable of targeting TSLP, respectively.

56. The multispecific molecule according to claim 55, wherein the two first binding domains capable of targeting IL-11 are linked directly or indirectly to the C-termini of two heavy chains of the second binding domain capable of targeting TSLP, respectively.

57. The multispecific molecule according to any one of claims 1-56, comprising a first polypeptide chain and a second polypeptide chain.

58. The multispecific molecule according to claim 57, wherein the first polypeptide chain comprises the first binding domain capable of targeting IL-11 and the heavy chain of the second binding domain capable of targeting TSLP.

59. The multispecific molecule according to any one of claims 58-59, wherein in the first polypeptide chain, said first binding domain capable of targeting IL-11 is linked to the N-termini of the heavy chain of said second binding domain capable of targeting TSLP directly or indirectly.

60. The multispecific molecule according to any one of claims 58-60, wherein in the first polypeptide chain, said first binding domain capable of targeting IL-11 is linked to the C-termini of the heavy chain of said second binding domain capable of targeting TSLP directly or indirectly.

61. The multispecific molecule according to any one of claims 57-60, wherein the first polypeptide chain comprises said first binding domain capable of targeting IL-11 and the heavy chain of said second binding domain capable of targeting TSLP linked by a linker.

62. The multispecific molecule according to any one of claims 57-61, wherein the first polypeptide chain comprises an amino acid sequence optionally selected from those as set forth in SEQ ID NO: 58, SEQ ID NO: 59, SEQ ID NO: 60, SEQ ID NO: 61, SEQ ID NO: 62, and SEQ ID NO: 63.

63. The multispecific molecule according to any one of claims 57-62, wherein the second polypeptide chain comprises a light chain of the second binding domain.

64. The multispecific molecule according to any one of claims 57-63, wherein the light chain of the second polypeptide chain comprises an amino acid sequence optionally selected from SEQ ID NO: 16, SEQ ID NO: 17 and SEQ ID NO: 18.

65. The multispecific molecule according to any one of claims 57-64, comprising two said first polypeptide chain and two said second polypeptide chain.

66. The multispecific molecule according to any one of claims 57-65, wherein the first polypeptide chain and the second polypeptide chain are linked by a disulfide bond.

67. A pharmaceutical combination comprising a first antibody capable of targeting IL-11 and a second antibody capable of targeting TSLP.

68. The pharmaceutical combination according to claim 67, wherein the first antibody and the second antibody are present as a mixture.

69. The pharmaceutical combination according to claim 67 or 68, wherein the first antibody and the second antibody are each present independently.

70. The pharmaceutical combination according to any one of claims 67-69, wherein the antibody is selected from the group consisting of a monoclonal antibody, a single chain antibody, a chimeric antibody, a multispecific antibody, a humanized antibody, a fully human antibody, a nanobody, and/or a heavy chain antibody.

71. The pharmaceutical combination according to any one of claims 67-70, wherein the first antibody comprises HCDR3, HCDR2 and HCDR1 of a heavy chain variable region VH; wherein the amino acid sequence of the HCDR3 is as set forth in SEQ ID NO: 7, the amino acid sequence of the HCDR2 is as set forth in SEQ ID NO: 6, and the amino acid sequence of the HCDR1 is as set forth in SEQ ID NO: 5.

72. The pharmaceutical combination according to any one of claims 67-71, wherein the first antibody comprises an antibody heavy chain variable region VH, and the amino acid sequence of the VH is as set forth in SEQ ID NO: 4.

73. The pharmaceutical combination according to any one of claims 67-72, wherein the heavy chain variable region is VHH.

74. The pharmaceutical combination according to any one of claims 67-73, wherein the first antibody targeting IL-11 comprises an antibody heavy chain constant region, said antibody heavy chain constant region derived from an IgG heavy chain constant region.

75. The pharmaceutical combination according to any one of claims 67-74, wherein the first antibody targeting IL-11 is a nanobody comprising an amino acid sequence as set forth in SEQ ID NO: 4.

76. The pharmaceutical combination according to any one of claims 67-70, wherein the first antibody targeting IL-11 can be an antibody known in the prior art.

77. The pharmaceutical combination according to any one of claims 67-70 and 76, wherein the first antibody targeting IL-11 comprises an antibody heavy chain variable region VH and/or an antibody light chain variable region VL.

78. The multispecific molecule according to any one of claims 67-70 and 76-77, wherein the first antibody targeting IL-11 comprises HCDR3, HCDR2 and HCDR1 of a heavy chain variable region VH; wherein the HCDR1 can be selected from sequences as set forth in SEQ ID NOs: 64, 65, 95, 104, 110 and 116, the HCDR2 can be selected from sequences as set forth in SEQ ID NOs: 66, 72, 73, 74, 96, 105, 111 and 117, and the HCDR3 can be selected from sequences as set forth in SEQ ID NOs: 67, 68, 97, 106, 112 and 118.

79. The multispecific molecule according to any one of claims 67-70 and 76-78, wherein the first antibody targeting IL-11 comprises LCDR3, LCDR2 and LCDR1 of a light chain variable region VL; wherein the LCDR1 can be selected from sequences as set forth in SEQ ID NOs: 69, 70, 71, 98, 101, 107, 113 and 119, the LCDR2 can be selected from sequences as set forth in SEQ ID NOs: 72, 73, 99, 102, 108, 114 and 120, and the LCDR3 can be selected from sequences as set forth in SEQ ID NOs: 74-94, 100, 103, 109, 115 and 121.

80. The multispecific molecule according to any one of claims 67-70 and 76-79, wherein the first antibody targeting IL-11 comprises an antibody heavy chain variable region VH, and the VH may comprise an amino acid sequence as set forth in SEQ ID NOs: 122-134.

81. The multispecific molecule according to any one of claims 67-70 and 76-80, wherein the first antibody targeting IL-11 comprises an antibody light chain variable region VL, and the VL may comprise an amino acid sequence as set forth in SEQ ID NOs: 135-177.

82. The multispecific molecule according to any one of claims 67-70 and 76-81, wherein the first antibody capable of targeting IL-11 comprises an antibody heavy chain constant region.

83. The multispecific molecule according to any one of claims 67-70 and 76-82, wherein the first antibody capable of targeting IL-11 comprises an antibody light chain constant region.

84. The pharmaceutical combination according to any one of claims 67-83, wherein the second antibody comprises HCDR3, HCDR2 and HCDR1 of a heavy chain variable region VH; wherein the amino acid sequence of the HCDR3 is as set forth in SEQ ID NO: 21, the amino acid sequence of the HCDR2 is as set forth in SEQ ID NO: 20, and the amino acid sequence of the HCDR1 is as set forth in SEQ ID NO: 19.

85. The pharmaceutical combination according to any one of claims 67-84, wherein the second antibody comprises an antibody heavy chain variable region VH, wherein the amino acid sequence of the VH is optionally selected from SEQ ID NO: 49, SEQ ID NO: 50, SEQ ID NO: 51, SEQ ID NO: 52, and SEQ ID NO: 53.

86. The pharmaceutical combination according to any one of claims 67-85, wherein the second antibody comprises LCDR1, LCDR2 and LCDR3 of a light chain variable region VL; wherein the amino acid sequence of the LCDR1 is as set forth in SEQ ID NO: 22, the amino acid sequence of LCDR2 is as set forth in SEQ ID NO: 23 (GAR), and the amino acid sequence of LCDR3 is as set forth in SEQ ID NO: 24.

87. The pharmaceutical combination of any one of claims 67-86, wherein the second antibody comprises an antibody light chain variable region VL, wherein the amino acid sequence of the VL is optionally selected from SEQ ID NO: 54, SEQ ID NO: 55, SEQ ID NO: 56, and SEQ ID NO: 57.

88. The pharmaceutical combination according to any one of claims 67-87, wherein the second antibody comprises an amino acid sequence selected from any one of the following groups of VH and VL:
1) VH: SEQ ID NO: 53, VL: SEQ ID NO: 57;
2) VH: SEQ ID NO: 49, VL: SEQ ID NO: 54;
3) VH: SEQ ID NO: 49, VL: SEQ ID NO: 55;
4) VH: SEQ ID NO: 49, VL: SEQ ID NO: 56;
5) VH: SEQ ID NO: 50, VL: SEQ ID NO: 54;
6) VH: SEQ ID NO: 50, VL: SEQ ID NO: 55;
7) VH: SEQ ID NO: 50, VL: SEQ ID NO: 56;
8) VH: SEQ ID NO: 51, VL: SEQ ID NO: 54;
9) VH: SEQ ID NO: 51, VL: SEQ ID NO: 55;
10) VH: SEQ ID NO: 51, VL: SEQ ID NO: 56;
11) VH: SEQ ID NO: 52, VL: SEQ ID NO: 54;
12) VH: SEQ ID NO: 52, VL: SEQ ID NO: 55; and
13) VH: SEQ ID NO: 52, VL: SEQ ID NO: 56.

89. The pharmaceutical combination according to claims 67-83, wherein the second antibody can be an antibody known in the prior art.

90. The pharmaceutical combination according to claims 67-83 and 89, wherein the second antibody is Tezepelumab.

91. The pharmaceutical combination according to any one of claims 67-83 and 89, wherein the second antibody capable of targeting TSLP comprises HCDR1, HCDR2 and HCDR3 of a heavy chain variable region VH; wherein the amino acid sequence of the HCDR1 is as set forth in SEQ ID NO: 178, the amino acid sequence of the HCDR2 is as set forth in SEQ ID NO: 179, and the amino acid sequence of the HCDR3 is as set forth in SEQ ID NO: 180.

92. The pharmaceutical combination according to any one of claims 67-83, 89 and 91, wherein the second antibody capable of targeting TSLP comprises LCDR3, LCDR2 and LCDR1 of a light chain variable region VL; wherein the amino acid sequence of the LCDR1 is as set forth in SEQ ID NO: 181, the amino acid sequence of the LCDR2 is as set forth in SEQ ID NO: 182, and the amino acid sequence of the LCDR3 is as set forth in SEQ ID NO: 183.

93. The pharmaceutical combination according to any one of claims 67-83, 89 and 91-92, wherein the second antibody capable of targeting TSLP comprises an antibody heavy chain variable region VH, and the VH comprises an amino acid sequence as set forth in SEQ ID NO: 184.

94. The pharmaceutical combination according to any one of claims 67-83, 89 and 91-93, wherein the second antibody capable of targeting TSLP comprises an antibody light chain variable region VL, and the VL comprises an amino acid sequence as set forth in SEQ ID NO: 185.

95. The pharmaceutical combination according to any one of claims 67-83, 89 and 91-94, wherein the second antibody capable of targeting TSLP comprises an antibody heavy chain constant region.

96. The pharmaceutical combination according to any one of claims 67-83, 89 and 91-95, wherein the second antibody capable of targeting TSLP comprises an antibody light chain constant region.

97. The pharmaceutical combination according to any one of claims 67-83 and 89, wherein the second binding domain capable of targeting TSLP comprises HCDR1, HCDR2 and HCDR3 of a heavy chain variable region; wherein the amino acid sequence of the HCDR1 is as set forth in SEQ ID NO: 186, the amino acid sequence of the HCDR2 is as set forth in SEQ ID NO: 187, and the amino acid sequence of the HCDR3 is as set forth in SEQ ID NO: 188.

98. The pharmaceutical combination according to any one of claims 67-83, 89 and 97, wherein the second binding domain capable of targeting TSLP comprises HCDR1, HCDR2 and HCDR3 of a heavy chain variable region; wherein the amino acid sequence of the HCDR1 is as set forth in SEQ ID NO: 189, the amino acid sequence of the HCDR2 is as set forth in SEQ ID NO: 190, and the amino acid sequence of the HCDR3 is as set forth in SEQ ID NO: 191.

99. The pharmaceutical combination according to any one of claims 67-83, 89 and 97-98, wherein the second binding domain capable of targeting TSLP comprises an antibody heavy chain variable region VH, and the VH comprises an amino acid sequence as set forth in SEQ ID NO: 192.

100. The pharmaceutical combination according to any one of claims 67-83, 89 and 99-100, wherein the second binding domain capable of targeting TSLP comprises an antibody heavy chain variable region VH, and the VH comprises an amino acid sequence as set forth in SEQ ID NO: 193.

101. One or more isolated nucleic acid molecule encoding the multispecific molecule of any one of claims 1-66.

102. A vector comprising the nucleic acid molecule according to claim 101.

103. A cell comprising the nucleic acid molecule according to claim 101 or the vector according to claim 102.

104. A method for preparing the multispecific molecule of any one of claims 1-66, comprising culturing the cell of claim 103 under the conditions that enable the expression of the multispecific molecule of any one of claims 1-66.

105. A pharmaceutical composition comprising the multispecific molecule of any one of claims 1-66, the pharmaceutical combination of any one of claims 67-100, the nucleic acid molecule of claim 101, the vector of claim 102, and/or the cell of claim 103, and optionally a pharmaceutically acceptable adjuvant.

106. A method for blocking the binding of TSLP to a receptor TSLPR/IL7Ra complex, comprising administering the multispecific molecule of any one of claims 1-66 and/or the pharmaceutical combination of any one of claims 67-100.

107. A method for inhibiting TARC (Thymus and Activation-Regulated Chemokine) production, comprising administering the multispecific antibody of any one of claims 1-66 and/or the pharmaceutical combination of any one of claims 67-100.

108. Use of the multispecific molecule of any one of claims 1-66, the pharmaceutical combination of any one of claims 67-100, the nucleic acid molecule of claim 101, the vector of claim 102, the cell of claim 103 and/or the pharmaceutical composition of claim 105 in preparing a medicament for preventing, alleviating and/or treating a disease or disorder.

109. The use according to claim 108, wherein the disease and/or disorder includes an IL-11 related disease.

110. The use according to any one of claims 108-109, wherein the disease and/or disorder includes a TSLP related disease.

111. The use according to any one of claims 108-110, wherein the disease and/or disorder includes a fibrotic disease, an inflammatory disease or a tumor.

112. A method for preventing, alleviating and/or treating a disease or disorder, comprising administering to a patient in need thereof the multispecific molecule of any one of claims 1-66, the pharmaceutical combination of any one of claims 67-100, the nucleic acid molecule of claim 101, the vector of claim 102, the cell of claim 103, and/or the pharmaceutical composition of claim 105.

113. The method according to claim 112, wherein the disease and/or disorder includes an IL-11 related disease.

114. The method according to any one of claims 112-113, wherein the disease and/or disorder includes a TSLP related disease.

115. The method according to any one of claims 112-114, wherein the disease and/or disorder includes a fibrotic disease, an inflammatory disease or a tumor.

116. The multispecific molecule of any one of claims 1-66, the pharmaceutical combination of any one of claims 67-100, the nucleic acid molecule of claim 101, the vector of claim 102, the cell of claim 103 and/or the pharmaceutical composition of claim 105, for use in preventing, alleviating and/or treating a disease or disorder.

117. The multispecific molecule, pharmaceutical combination, nucleic acid molecule, vector, cell and/or pharmaceutical composition of claim 116, wherein the disease and/or disorder includes an IL-11 related disease.

118. The multispecific molecule, pharmaceutical combination, nucleic acid molecule, vector, cell and/or pharmaceutical composition of any one of claim 116 or 117, wherein the disease and/or disorder includes a TSLP related disease.

119. The multispecific molecule, pharmaceutical combination, nucleic acid molecule, vector, cell and/or pharmaceutical composition of any one of claims 116-118, wherein the disease and/or disorder includes a fibrotic disease, an inflammatory disease or a tumor.
